(19) Europäisches Patentamt
European Patent Office
Office européen des brevets

(11) **EP 2 207 507 B1**

(12) **EUROPEAN PATENT SPECIFICATION**

(45) Date of publication and mention
of the grant of the patent:
**21.11.2018 Bulletin 2018/47**

(21) Application number: **08839155.2**

(22) Date of filing: **15.10.2008**

(51) Int Cl.:
*A61K 9/70* (2006.01)     *A61K 31/4468* (2006.01)

(86) International application number:
**PCT/US2008/080029**

(87) International publication number:
**WO 2009/052204 (23.04.2009 Gazette 2009/17)**

(54) **ONCE-A-DAY REPLACEMENT TRANSDERMAL ADMINISTRATION OF FENTANYL**

EINMAL TÄGLICHE TRANSDERMALE ERSATZ-VERABREICHUNG VON FENTANYL

ADMINISTRATION TRANSDERMIQUE À REMPLACEMENT QUOTIDIEN DE FENTANYLE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **15.10.2007 US 979911 P**

(43) Date of publication of application:
**21.07.2010 Bulletin 2010/29**

(73) Proprietor: **Alza Corporation**
**Vacaville, CA 95688 (US)**

(72) Inventors:
• **HWANG, Stephen, S.**
 **Palo Alto**
 **CA 94303 (US)**
• **GALE, Robert, M.**
 **Los Altos**
 **CA 94024 (US)**

(74) Representative: **Oates, Edward Christopher et al**
**Carpmaels & Ransford LLP**
**One Southampton Row**
**London WC1B 5HA (GB)**

(56) References cited:
**WO-A1-02/074286**     **US-A- 5 186 939**
**US-A- 5 186 939**     **US-A1- 2003 178 031**
**US-A1- 2005 208 117**     **US-A1- 2005 208 117**

## Description

Technical Field

The present disclosure relates to delivery devices for the transdermal administration of fentanyl base.

Background

Opioid drugs are known and are included in various drug products. For example, fentanyl and analogs thereof, such as alfentanil, carfentanil, lofentanil, remifentanil, sufentanil, trefentanil and the like, are powerful synthetic opioids which have demonstrated utility in both human and veterinary medicine. In human medicine, alfentanil, fentanyl, remifentanil and sufentanil have been granted regulatory approval for use as general anesthetics. Transdermal administration of fentanyl and analogs thereof for the treatment of both acute and chronic pain has been suggested in patents and published applications (See e.g., U.S. Patent Nos. 4,466,953; 4,470,962; 4,588,580; 4,626,539; 5,006,342; 5,186,939; 5,310,559; 5,474,783; 5,656,286; 5,762,952; 5,948,433; 5,985,317; 5,958,446; 5,993,849; 6,024,976; 6,063,399 and 6,139,866, and U.S. Application Nos. 2003002682, 20050208117, 2002119187, and 20040234584). Moreover, products containing fentanyl, including a fentanyl-containing transdermal patch, have also been marketed for analgesia in the treatment of chronic pain.

Examples of transdermal patch configurations include monolithic and multilayer devices. A monolithic device is relatively simple and may be characterized by an adhesive monolith including a drug-containing reservoir disposed on a backing. The drug-containing reservoir in such a device is typically formed from a pharmaceutically acceptable pressure sensitive adhesive. In some cases, the drug-containing reservoir can be formed from a non-adhesive material, the skin-contacting surface of which is provided with a

thin layer of a suitable adhesive. The rate at which the drug is administered to the patient from these patches can vary due to normal person-to-person and skin site-to-skin site variations in the permeability of skin to the drug. A multilayer, or multilaminate device, may include a matrix drug reservoir or a liquid reservoir bound by one or more membranes. For example, in a multilaminate patch, a drug release-rate controlling membrane may be disposed between the drug reservoir and the skin-contacting adhesive. By controlling the release rate of drug from the patch, a release-rate controlling membrane serves to reduce the effects of variations in skin permeability.

In addition to monolithic and multilayer designs, the drug-containing reservoirs of transdermal patches can have the drug either completely dissolved in the reservoir (subsaturated patches, see e.g., U.S. Pat. Nos., 4,704,282; 4,725,439; 4,867,982; 4,908,027; 5,004,610; 5,152,997; 5,164,190; 5,342,623; 5,344,656; 5,364,630; 5,462,745; 5,633,008 and 6,165,497) or can contain an excess of undissolved drug over the saturation concentration (depot patches). Because transdermal patches deliver a drug by diffusion through the skin, the delivery rate of the drug from the patch is generally governed by Fick's law and is typically proportional to the level of saturation of the drug in the reservoir.

A fentanyl product that has been available widely as a transdermal patch for analgesia is the DURAGESIC® patch. See, for example, the labeling describing this patch and its use in, e.g., Physicians Desk Reference, 58th Edition, 2004, pages 1751-1755. Another fentanyl patch with fentanyl dissolved in a pressure sensitive adhesive, DUROGESIC® DTRANS® (or DUROGESIC® SMAT) matrix patch, is available in certain countries as a transdermal patch for analgesia, see, Summary of Product Characteristics of "DUROGESIC® DTRANS® transdermal patch" and DUROGESIC ® DTRANS® transdermal patch "Patient Information Leaflet" accessible by Internet to the public at http://emc.medicines.org.uk. The DUROGESIC® DTRANS® transdermal patch is a product that administers fentanyl for 3 days and is indicated for the treatment of chronic pain, as opposed to post-operative or other acute pain. The DUROGESIC® DTRANS® matrix fentanyl patch is intended to be sequentially removed and replaced with a fresh patch applied to a new skin site at the end of each 3 day period to provide relief from chronic pain and it is contemplated that doses may be increased over time and that concurrent use of other analgesics may occur to deal with breakthrough pain.

Summary

The present invention provides transdermal patches and kits for transdermal delivery of fentanyl base. It provides a once-a-day patch (1-day patch) for transdermal delivery of fentanyl base at a targeted rate and in an amount sufficient to induce and maintain analgesia over a period of treatment that lasts about one day. In each embodiment, transdermal patches as described herein may be prepared for administration to human patients.

In one aspect, the invention provides a transdermal patch for administration of fentanyl base through the skin, comprising:

a backing layer;
a reservoir disposed on the backing layer, at least the skin contacting surface of the reservoir being adhesive; the reservoir comprising a polymeric composition containing polyacrylate and an amount of fentanyl base sufficient to induce and maintain analgesia in a human for one day,
wherein the normalized area based on nominal dose strength is 0.2 to 0.4 $cm^2$ per $\mu g/h$ per day and the fentanyl loading per surface area is 0.14 to 0.3 $mg/cm^2$.

In another aspect, the invention provides a kit for administering a drug with a transdermal patch, comprising:

(a) a transdermal patch for administration of fentanyl base through the skin, comprising:

a backing layer;
a reservoir disposed on the backing layer, at least the skin contacting surface of the reservoir being adhesive;
the reservoir comprising a polymeric composition containing polyacrylate and an amount of fentanyl base sufficient to induce and maintain analgesia in a human for one day; and

(b) an instruction print including instruction on daily replacement of the transdermal patch,
wherein the normalized area based on nominal dose strength is 0.2 to 0.4 $cm^2$ per $\mu g/h$ per day and the fentanyl loading per surface area is 0.14 to 0.3 $mg/cm^2$.

In addition to transdermal patches, the present disclosure provides fentanyl base for use in a method of treating pain, the method comprising replacing one monolithic transdermal patch daily on the skin of a human in need thereof, wherein the patch contains a backing and a reservoir comprising a polymeric composition containing polyacrylate and fentanyl base, wherein the normalized area based on nominal dose strength is 0.2 to 0.4 $cm^2$ per $\mu g/h$ per day and the fentanyl loading per surface area is 0.14 to 0.3 $mg/cm^2$. In an example of such an embodiment, the method includes applying to a patient at least one patch designed to deliver one or more of fentanyl or an analog thereof, followed by removal and replacement of at least one of said at least one patch more often than every 3 days, for example, more often than every two days, or alternatively, every day.

Brief Description of the Drawings

The present disclosure is illustrated by way of examples in embodiments and not limitation in the figures of the accompanying drawings in which like references indicate similar elements. The figures are not shown to scale unless indicated otherwise in the content. In the graphs, vertical lines connected to data points indicate standard deviations.

FIG. 1 illustrates a schematic sectional view through one embodiment of a transdermal therapeutic system.

FIG. 2 illustrates a schematic sectional view through another embodiment of transdermal therapeutic system.

FIG. 3 illustrates the comparison of blood drug concentration of a simulation of the once-a-day application of 1-day transdermal fentanyl patches versus the once-every-3-days application of 3-day transdermal fentanyl patches at steady state.

FIG. 4 illustrates the comparison of blood drug concentration of a simulation of the once-a-day application of 1-day transdermal fentanyl patches versus the actual experimental data of 3-days application of 3-day transdermal fentanyl patches.

FIG. 5 illustrates another comparison of blood drug concentration of a simulation of the once-a-day application of 1-day transdermal fentanyl patches versus the actual experimental data of 3-day application of 3-day transdermal fentanyl patches.

FIG. 6 represents actual serum fentanyl concentrations collected over time in using 3-day patches of different dose strengths.

FIG. 7 illustrates the serum fentanyl concentration experimental results of administering 3-day fentanyl transdermal patches with daily replacement by a new patch.

FIG. 8 shows the summary of the averaged data (over the subjects) on the fentanyl concentration in the blood of the subjects for various thicknesses of matrix drug layers.

FIG. 9 shows the simulated data of fentanyl concentration by repeated application of 1-day patch versus 3-day patches up to 336 hours.

FIG. 10 shows the summary of the averaged data (over the subjects) on the serum fentanyl concentration in the subjects from the 216th hour to the 288th hour for the 1-day patches compared with the 3-day patches.

FIG. 11 shows the comparison of the steady state 3-day patch data of FIG. 10 versus the simulated steady state 3-day patch data scaled from the data of FIG. 9.

FIG. 12 shows the comparison of the steady state 1-day patch data of FIG. 10 versus the simulated steady state 1-day patch data scaled from the data of FIG. 9.

FIG. 13 shows the data on serum fentanyl concentration for 1-day fentanyl patches of different dose strengths applied for one day.

FIG. 14 shows the summary of the averaged data (over subjects) on the fentanyl concentration in the blood of the subjects at steady state of application of an embodiment of the 1-day patches versus 3-day patches.

FIG. 15 shows the summary of the averaged data (over subjects) on the fentanyl concentration in the blood of the subjects at steady state of application of another embodiment of the 1-day patches versus 3-day patches.

FIG. 16 is a graph showing the serum concentration of fentanyl and its metabolite norfentanyl in one subject with the administration of fentanyl by 1-day patches.

Detailed Description

Transdermal patches for the delivery of fentanyl base are described herein. Methods for delivery of opioid drugs are also disclosed, and in specific embodiments, the methods include applying one or more transdermal patches as described herein to a patient. In some embodiments, the present disclosure provides daily replacement 1-day (sometimes called "QD" for convenience) fentanyl patches for analgesia. In specific embodiments, daily replacement application of one patch, as described herein, on the skin of a patient may achieve a steady state plasma level within a therapeutically effective range. Thus, if there is only one patch on the skin, daily replacement of the patch will establish a steady state plasma profile within a therapeutically effective range.

It has been found that when multi-day transdermal patches for delivery of fentanyl, such as the DUROGESIC® DTRANS® matrix patches, where applied with replacement done once per day, instead of every three days, the plasma concentration of fentanyl increased rapidly over the first three doses, resulting in drug plasma levels that were above the steady state level achieved when the same patch is replaced every three days. Without being limited to or bound by a particular theory, it is thought that this performance is achieved because, at replacement, although a used patch is removed the old and the new patch are not present on the skin simultaneously, a certain amount of the drug from the old patch is held in the tissue of the user and requires a significant amount of time for it to be cleared from the user, resulting in a depot effect. For instance, it has been found that the half-life of the transdermal delivered fentanyl is quite long relative to that for intravenously administered fentanyl due to the depot effect.

For a patch that is replaced only every 3 or 4 days, the depot effect upon plasma fentanyl concentration accumulation may be relatively unnoticeable. However, it has been found that when a 3-day patch is applied on a patient with daily replacement, a higher level of the drug in the plasma is achieved than when the 3-day patch is applied every 3 or 4 days. Thus, it has been found that a transdermal patch according to the present invention that is applied on a patient with daily replacement may be relatively smaller than

and/or loaded with relatively less drug than a multi-day patch, such as a 3-day patch, while still achieving and maintaining therapeutically effective drug plasma levels. In particular, it has been found that, in order to reach or maintain a plasma concentration of fentanyl base targeted by a multi-day transdermal patch, a patch system smaller than currently available multi-day patches, such as currently available 3-day transdermal fentanyl patches, may be used. In some embodiments, a 1-day patch according to the present description includes half, or less, the drug loading of prior 3-day patches, yet may be used to achieve and/or maintain targeted, therapeutically effective opioid drug plasma levels when applied with daily replacement. Even further, we have found that targeted and therapeutically effective fentanyl plasma levels can be achieved and/or maintained by daily application and replacement of a single transdermal patch as described herein. Of course, it is contemplated that, even when only a single patch is applied and replaced daily, there may be some overlap of time where more than one patch is still applied to a patient, e.g., for a few seconds or even minutes during replacement, without leading to a significant difference in drug absorbed.

The present disclosure provides transdermal patches that allow users to deliver and maintain therapeutic levels of opioid drugs without exceeding a targeted $C_{max}$. Moreover the transdermal patches described herein provide desirable steady state delivery of opioid drugs, with narrow fluctuations between $C_{max}$ and $C_{min}$ of the opioid delivered. The control in fluctuation of $C_{max}$ and $C_{min}$ of the drug delivered by the patches described herein works to maintain therapeutic effect and potential side effects of the drug. In embodiments of the transdermal patch described herein, the patch is a 1-day patch that delivers opioid drug such that the difference between $C_{max}$ and $C_{min}$ provided by the 1-day patch is smaller than that achieved by a multi-day patch designed for delivery of the same opioid drug.

The patches described herein exhibit higher wt% utility of drug per day relative to multi-day patches. For example, the patches described herein are formulated and configured for delivery of fentanyl base and provide higher wt% utility of drug per day as compared to multi-day patches with the same drug, such as in the DUROGESIC® DTRANS® matrix patch. In some embodiments, the patches described herein may contain a lower content of fentanyl base than presently available products or multi-day opioid patches. In some embodiments, the present disclosure provides 1-day patches that provide a percentage utilization of fentanyl base after 1 day that approximates the percent utilization of opioid drug provided by multi-day patches after multiple days of use. In one such embodiment, a 1-day fentanyl base patch is described that provides a percentage utilization of fentanyl after one day of use that approximates the percent utilization of fentanyl provided by 3-day fentanyl matrix patches after three days of use. The relatively lower drug loading and high wt% utility of drug provided by embodiments of the patches described herein can lead to less residual drug remaining in the patch after use, and such characteristics may reduce the risk that patches according to such embodiments may be subject to abuse or illicit use.

The 1-day patches described herein may also afford increased convenience for users and lead to increased patient compliance. In some instances, it may be desirable to remove a transdermal patch on a daily basis, at least for a brief period of time. For example, where a multi-day opioid patch is used, it may be desirable to remove such patch while bathing, as submersion or exposure to large amounts of water may lead to some amount of drug passing out of the patch. Further, human beings are creatures of habit, and the routine of daily replacement can be more conducive for compliance than replacement that is done every three or four days.

A transdermal patch according to the present description includes a backing layer and a drug reservoir disposed on the backing layer. At least the skin-contacting surface of the reservoir is adhesive, with the reservoir including a polymeric composition containing an amount of fentanyl base sufficient to induce and maintain analgesia in a patient for about one day. In such embodiments the drug reservoir may or may not contain undissolved fentanyl base. Therefore, in some embodiments, the drug reservoir includes no undissolved fentanyl base and, optionally, no undissolved material in the drug reservoir adhesive. Alternatively, in other embodiments, a drug reservoir of a transdermal patch as described herein may include undissolved fentanyl base.

The patches described herein may be constructed and formulated for administration to human patients.

The present disclosure provides a non-rate controlled, monolithic patch without a rate-controlling membrane for transdermal delivery of fentanyl base at an administration rate sufficient to induce and maintain analgesia by daily replacement of a patch every day. The fentanyl is the base form of fentanyl (the non-ionized form of fentanyl being present in the reservoir). The patch includes a backing layer and a drug reservoir disposed on the backing layer. The skin-contacting surface of the drug reservoir is adhesive and the reservoir contains a polymeric composition containing polyacrylate with or without undissolved components containing an amount of fentanyl base sufficient to induce and maintain analgesia in a human for one day. Such an embodiment may be utilized in a method whereby a single such patch is administered with daily replacement and achieves a steady state plasma level within a therapeutically effective range.

The transdermal patches of the present disclosure may be used in methods of administering fentanyl base to a subject in need thereof. In one embodiment, a method described herein includes applying then replacing one transdermal patch on a daily basis on the skin of the subject such that a steady state plasma profile within a therapeutic range is achieved. The drug delivered by the patch is fentanyl, which is present in the reservoir in (un-ionized) base form, and may be dissolved in a polyacrylate adhesive. In certain embodiments, undissolved fentanyl may be present. The subject in need of opioid administration may be a human subject.

In another embodiment, the transdermal pactches of the present disclosure may be used in a method of administering fentanyl base to a subject in need thereof by applying two or more patches initially on day one and subsequently daily applying a new patch to replace a day-old patch starting from day two, such that a plasma profile of fentanyl within a therapeutic range is achieved in one day. In such an embodiment, some of the two or more patches can be removed on days following the first day and not replaced such that at steady state, only one patch remains applied on the subject's skin at a given time for analgesia. The patches utilized contain a backing layer and a drug reservoir having a polymeric composition containing polyacrylate and the fentanyl base. The drug delivered by the patches is fentanyl, which is present in the reservoir in (un-ionized) base form, and may be dissolved in a polyacrylate adhesive. In certain embodiments, undissolved fentanyl may be present. The subject in need of fentanyl administration may be a human subject.

In further embodiments, the transdermal patches of the present invention may be used in methods that include an additional step determining whether the patient is a narcotic

opioid tolerant individual or narcotic opioid naive. Such information may be obtained, for example, based on the patient's medical record, and may then be used in determining the dosing or application regimen that may be recommended for the individual. For example, if a patient is opioid tolerant, in order to provide effective pain management, it may be necessary raise the level of opioid drug in the blood to a targeted steady state level relatively quickly. In such an instance, administering two or more patches to the skin of the individual initially, followed by subsequently reducing the number of patches on the skin can quickly bring the level of opioid drug up to a therapeutic, steady state level. Alternatively, if a patient is determined to be opioid naive, such a patient may benefit from a relatively lower concentration of opioid in the blood. An opioid naive patient may, therefore, benefit from administration of only a single patch initially, followed by daily replacement. In certain cases with opioid naive patients, achieving a steady state target plasma concentration in 2 to 3 days may be determined to be desirable. The regimen of starting initially by applying only one patch on day 1 will allow a gradual increase to occur and yet achieve acceptable analgesia due to the opioid-naive nature of the patient. As used herein, an "opioid naive" patient is one that has not been exposed sufficiently to the drug to develop any noticeable tolerance to the drug. As used herein, an "opioid tolerant" patient is one that has been exposed to the drug to such an extent that some noticeable tolerance to the drug has developed.

The present invention provides a kit that contains a transdermal patch together with an instruction print. The instruction print may be an insert or provided on a container or packaging and provides a user with instructions as to use. For example, the instruction print may describe how each patch is to be applied, the length of time each patch is to be applied to a patient, and how often each patch is to be removed and replaced with a new patch.

Definitions

In describing the present technology, the following terms will be employed, and are defined as indicated below. As used in this specification and the appended claims, the singular forms "a," "an" and "the" include plural references unless the text content clearly dictates otherwise.

As used herein, the term "transdermal" refers to the use of skin, mucosa, and/or other body surfaces as a portal for the administration of drugs by topical application of the drug thereto for passage into the systemic circulation.

As used herein, the term "drug" refers to any material that is intended to produce some biological, beneficial, therapeutic, or other intended effect, such as relief of symptoms of health disorder, but not agents (such as permeation enhancers) the primary effect of which is to aid in the delivery of another biologically active agent such as the therapeutic agent transdermally.

As used herein, the term "therapeutically effective" refers to the amount of drug or the rate of drug administration needed to produce the desired therapeutic result.

As used herein, the term "permeation enhancement" intends an increase in the permeability of skin to a drug in the presence of a permeation enhancer as compared to permeability of skin to the drug in the absence of a permeation enhancer. A "permeation-enhancing amount" of a permeation-enhancer is an amount of the permeation enhancer sufficient to increase the permeability of the body surface of the drug to deliver the drug at a therapeutically effective rate.

As used herein, "acrylate", "polyacrylate" or "acrylic polymer", when referring to a polymer for an adhesive or proadhesive, refers to polymer or copolymer of acrylic acid, ester(s) thereof, acrylamide, or acrylonitrile. Unless specified otherwise, it can be a homopolymer, copolymer, or a blend of homopolymers and/or copolymers.

As used herein, the term "an analog of fentanyl" (hereafter referred to as "analog") refers to potent and effective analgesics such alfentanil, carfentanil, lofentanil, remifentanil, sufentanil, trefentanil, and the like. An exemplary form is the base form of fentanyl or the analog.

As used herein, the term "subsaturated patch" refers to a patch wherein the concentration of the drug is below its solubility limit. "Saturated patch" refers to a formulation containing dispersed drug (e.g., fentanyl base) solid or liquid, at a concentration above the saturation concentration in the reservoir.

As used herein, the term "single phase polymeric composition" refers to a composition in which the drug and all other components are solubilized in a polymer and are present at concentrations no greater than, such as less than, their saturation concentrations in the reservoir, such that there are no undissolved components present in the composition over a substantial portion of the administration period; wherein all the components in combination with the polymer form a single phase.

As used herein, the term "component" refers to an element within the drug reservoir, including, but not limited to, a drug as defined above, additives, permeation enhancers, stabilizers, dyes, diluents, plasticizers, tackifying agents, pigments, carriers, inert fillers, antioxidants, excipients, gelling agents, anti-irritants, vasoconstrictors and the like.

As used herein, a "rate controlling membrane" refers to a drug release-rate controlling membrane that is disposed between the drug containing reservoir and the body surface, functioning to control the rate of the drug transfer from the reservoir into the body surface. An "un-rate-controlled" fentanyl patch means a patch without a rate control membrane.

A "DURAGESIC® fentanyl patch" refers to a fentanyl patch as discussed above (see also Physicians Desk Reference, 58th Edition, 2004, pages 1751-1755). A "DUROGESIC® SMAT matrix patch" and "DUROGESIC® DTRANS® transdermal patch" refers to a transdermal delivery patch of fentanyl in a polyacrylate matrix made available in Germany and the United Kingdom, respectively by Janssen-Cilag, see, Summary of Product Characteristics of "DUROGESIC® DTRANS® transdermal patch" and DUROGESIC® DTRANS ® transdermal patch "Patient Information Leaflet", which are publicly available.

The term "AUC" means the area under the curve obtained in a subject by plotting serum concentration of the beneficial agent in the subject against time, as measured from the time of start of dosing, to a time "t" after the start of dosing. $AUC_{inf}$ is the area under the curve extended to time of infinity. For steady state, the $AUC_{ss}$ is the area under the curve for a dose period for doses administered to time infinite. The AUC can be obtained by assaying serum samples from a patient. AUC can also be obtained for the serum fentanyl concentration profiles constructed by simulation based on data obtained from experiments. $AUC_{ss}$ and $AUC_{inf}$ are expected to be identical when the drug kinetics is linear. For transdermal fentanyl product, $AUC_{ss}$ and $AUC_{inf}$ have been demonstrated to be bioequivalent (Sathyan, et al "Evaluation of the bioequivalence of two transdermal fentanyl systems following single and repeat applications" Current Medical Research and Opinion 21(12) 1961-1968, 2005).

As used herein, the term "$C_{max}$" refers to the peak blood or plasma concentration of the drug, e.g., fentanyl or the analog thereof.

As used herein, the term "$C_{min}$" refers to the valley blood or plasma concentration of the drug, e.g., fentanyl or the analog thereof.

As used herein, the term "normalized $C_{max}$ (ng/ml/(mg/h))" refers to the $C_{max}$ (ng/ml) divided by the nominal rate of the drug administered (mg/h). Likewise, normalized $C_{min}$ corresponds to $C_{min}$ in a similar way. The nominal rate of drug administered is the average rate of drug administration the product is designed to deliver during the period of use (typically the rate stated in the product label).

As used herein, the term "bioavailability", refers to the rate and extent to which the active ingredient or active moiety is absorbed from a drug product and becomes available at the site of action. The rate and extent are established by the pharmacokinetic-parameters, such as, the peak blood or plasma concentration ($C_{max}$) of the drug and the area under the blood or plasma drug concentration-time curve (AUC).

Two different products are considered to be "pharmacologically equivalent" if they produce substantially the same therapeutic effects when studied under similar experimental conditions, as demonstrated through several *in vivo* and *in vitro* methods as described in greater detail hereinafter. Therapeutic effects depend on various factors, such as, potency of the drug, the concentration of the drug in the drug reservoir, the solubility and diffusivity of the drug in the skin, thickness of the skin, concentration of the drug within the skin application site, and the like, as described in greater detail hereinafter. In general, pharmacological equivalence is demonstrated using measures such as the area under the curve (AUC).

The AUC, $C_{max}$ and $C_{min}$ are parameters that are related to the characteristics of a patch. The peak blood or plasma concentration of the drug normalized for the rate of drug administered (i.e. normalized $C_{max}$ as defined below) is a parameter that relates to the characteristics of the patch.

When comparing two different products with the same drug, bioequivalence may be established by similar AUC and $C_{max}$ values per regulatory agency (such as the FDA) bioequivalence guidance.

Transdermal Patches

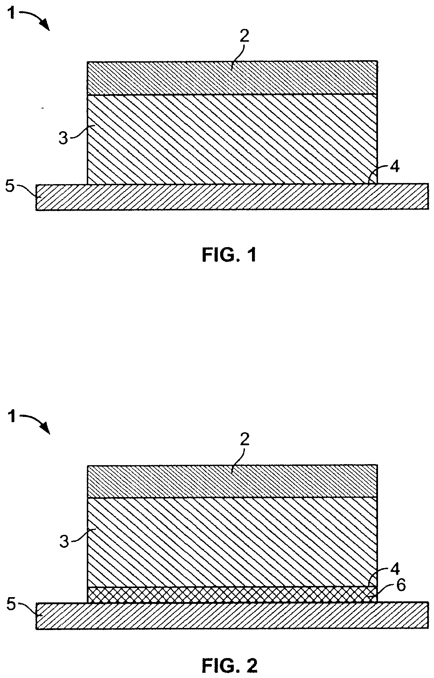

Exemplary transdermal drug delivery systems are illustrated by the embodiments shown in FIGS. 1 and 2. As shown in FIGS. 1 and 2, an embodiment of the transdermal monolithic patch 1 has a backing layer 2, a drug reservoir 3 disposed on the backing layer 2, and a peelable protective layer 5. In the reservoir 3, which can be a layer, at least the skin-contacting surface 4 is adhesive. The reservoir is a matrix (carrier) that is suitable for carrying the pharmaceutical agent (or drug) for transdermal delivery. Unless it is clear in the content to be otherwise, a "matrix" can be meant to refer to the carrier material with or without other ingredients such as the drug. In one embodiment, the whole matrix, with drugs and other optional ingredients, is a material that has the desired adhesive properties. The reservoir 3 can be either a single phase polymeric composition or a multiple phase polymeric composition. In a single phase polymeric composition the drug and all other components are present at concentrations no greater than, and preferably less than, their saturation concentrations in the reservoir 3. This produces a composition in which all components are dissolved (i.e., in the polymeric adhesive in the reservoir). It is to be understood that in other embodiments, the reservoir may contain drug solid or liquid and is above the solubility concentration. The reservoir 3 is formed using a pharmaceutically acceptable polymeric material that can provide acceptable adhesion for application to the body surface. In a multiple phase polymeric composition, at least one component, for example, a therapeutic drug that is present in amount more than the saturation concentration. In some embodiments, more than one component, e.g., a drug and a permeation enhancer or polymeric material, is present in amounts above the saturation concentration. In the embodiment shown in FIG. 1, the adhesive acts as the reservoir and includes a drug.

In the embodiment shown in FIG. 2, the skin-contacting surface of the reservoir 4 may be formulated with a thin adhesive coating 6. The reservoir 3 may be a single phase polymeric composition or a multiple phase polymeric composition as described earlier. The adhesive coating can contain the drug and permeation enhancer, as well as other ingredients.

The backing layer 2 may be formed from any material suitable for making transdermal delivery patches, such as a breathable or occlusive material, including fabric or sheet, made of polyvinyl acetate, polyvinylidene chloride, polyethylene, polyurethane, polyester, ethylene vinyl acetate (EVA), polyethylene terephthalate, polybutylene terephthalate, coated paper products, aluminum sheet and the like, or a combination thereof. In some embodiments, the backing layer includes low density polyethylene (LDPE) materials, medium density polyethylene (MDPE) materials or high density polyethylene (HDPE) materials, e.g., SARANEX (Dow Chemical, Midland, Mich.). The backing layer may be a monolithic or a multilaminate layer. In some embodiments, the backing layer is a multilaminate layer including nonlinear LDPE layer/linear LDPE layer/nonlinear LDPE layer. The backing layer can have a thickness of about 0.012 mm (0.5 mil) to 0.125 mm (5 mil); such as about 0.018 mm (0.75 mil) to 0.1 mm (4 mil); or alternatively about 0.025mm (1 mil) to 0.0875 mm (3.5 mil).

The adhesive reservoir 3 or the adhesive coating 6 may be formed from standard pressure sensitive polyacrylate adhesives known in the art.

As mentioned above, the drug reservoir 3 is disposed on the backing layer 2 and at least the skin-contacting surface of the reservoir 3 is adhesive. As mentioned, the skin-contacting surface can have the structure of a layer of adhesive. However, the whole reservoir 3 may be substantially of the same composition, without local variation or stratification, adhered to a peelable protective layer 5. The reservoir 3 may be formed from drug (or biologically active agent) reservoir materials as known in the art. For example, the drug reservoir may be formed from a polyacrylate-containing polymeric material in which the drug has reasonable solubility for the drug to be delivered within the desired range. In some embodiments, the reservoir 3 is formed from a pharmaceutically acceptable polyacrylate-containing adhesive, such as acrylate copolymer-based, as described in greater detail below. With the drug and optionally, other ingredients incorporated therein, the drug reservoir has the requisite adhesive property to retain the transdermal patch on the skin for the period of delivery. The drug reservoir or the matrix layer can have a thickness of about 0.2mils (0.005mm) to less than 4 mils (0.1 mm), such as about 0.5-1.5 mils (0.0125-0.0375 mm), or about 0.5-1.25 mil (0.0125 - 0.03 mm), or alternatively about 0.8-1.2 mil (0.02-0.03mm), or further about 0.9-1.1 mil (0.023-0.028mm). Generally, the thickness is thinner than the commercially marketed analgesic patches, which are about 2 mils.

The thickness of the reservoir is selected such that the desired flux (drug delivered in $\mu g/cm^2 h$) is achieved by a patch with a regimen of replacement once a day (about every 24 hours). The flux is dependent on diffusion and diffusion is a function of the concentration difference between the skin and the reservoir. The rate of drug delivered (in $\mu g/h$) for each patch is also dependent on the area of contact between the reservoir and the skin. Thus, the dimensions (including area and thickness) of the reservoir are selected to produce the desired flux and rate of drug delivery.

Another factor that may be considered in selecting the dimensions of the reservoir is the reduction or deterrence of abuse. If a potential abuser finds that only a relatively small amount of drug is available from a single patch, the potential abuser may be less likely to abuse the patch. Therefore, a patch having a relatively smaller reservoir volume and drug load may work to reduce or deter abuse. In one embodiment, a patch that includes a reservoir formed using PIB materials is provided. Reservoirs formed using PIB materials may be desirable because they can provide comparable skin flux at reduced fentanyl content due to the lower solubility of fentanyl in PIB.

For aesthetic reasons, it may be desirable to reduce the surface area of the patch, and therefore that of the reservoir. In some embodiments, the surface area of the patch (and that of the reservoir) is significantly smaller than that of a patch designed for multi-day use. For example, in one such embodiment, the surface area of a patch described herein is significantly smaller than the surface area of a 3-day patch designed for delivery of the same opioid drug. The surface area of the patch, i.e., of the reservoir, will be described below.

In the context of abuse reduction or deterrence, drug loading (e.g., the concentration and/or the total amount of amount of drug loaded into a patch) may also be considered. In specific embodiments, transdermal patches for the administration of fentanyl base include a drug reservoir wherein the total amount of drug loaded in the drug reservoir may be about less than 12 mg for a 100 $\mu g/h$ dose strength; such as about less than 8 mg for a 100 $\mu g/h$ dose strength; or about 7.5mg or less for a 100 $\mu g/h$ dose strength. For a devices of other dose strengths (other than 100 $\mu g/h$) of the same material but difference in size, the drug content in the patch can be adjusted proportionally to size (area), i.e., if the only difference is area (typically called size), then the scaling is proportional to the area (i.e., size). For example, a 50 $\mu g/h$ dose strength would have half of the above-described drug content of that of a 100$\mu g/h$ dose strength by having half the area thereof.

Yet another factor that may be considered in the context of abuse reduction or deterrence is the amount of residual drug (drug remaining in the patch after use) contained in a used patch. For example, if the wt% utility of the drug in the patch (when the patch is removed after the period of use) is increased, the amount of drug available for potential abuse after use of the patch is reduced, and the potential of abusing such a device may be reduced. In specific embodiments, a patch as described herein may include a drug reservoir having dimensions selected to achieve an amount of residual fentanyl base of about less than 6 mg, or less, for a 100 μg/h dose strength. In one such embodiment, the amount of residual fentanyl base is about 5 mg, or less.

It is to be understood that the above parameters are interrelated and it is no simple matter to set about reducing the amount of fentanyl base loading, reducing the concentration of the fentanyl base, and reducing the size of the reservoir, while enhancing, for example, the wt% utility of the drug in the patch and providing a patch capable of delivering fentanyl base over a desired period of time in a manner that achieves therapeutic effect in the patient. As detailed herein, the present disclosure provides embodiments of patches that, relative to multi-day systems, include less fentanyl base and may make the fentanyl base contained therein less available for abuse before and/or after use.

The adhesive reservoir 3 or the adhesive coating 6 included in a patch as described herein, may be formed from standard pressure sensitive polyacrylate adhesives known in the art.

Polyacrylates (acrylic polymers) may be comprised of a copolymer or terpolymer comprising at least two or more exemplary components selected from the group comprising acrylic acids, alkyl acrylates, methacrylates, copolymerizable secondary monomers or monomers with functional groups. Examples of monomers include, but are not limited to, vinyl acetate, acrylic acid, methacrylic acid, methoxyethyl acrylate, methyl acrylate, ethyl acrylate, butyl acrylate, butyl methacrylate, hexyl acrylate, hexyl methacrylate, 2-ethylbutyl acrylate, 2-ethylbutyl methacrylate, isooctyl acrylate, isooctyl methacrylate, 2-ethylhexyl acrylate, 2-ethylhexyl methacrylate, decyl acrylate, decyl methacrylate, dodecyl acrylate, dodecyl methacrylate, tridecyl acrylate, tridecyl methacrylate, hydroxyethyl acrylate, hydroxypropyl acrylate, acrylamide, dimethylacrylamide, acrylonitrile, dimethylaminoethyl acrylate, dimethylaminoethyl methacrylate, tert-butylaminoethyl acrylate, tert-butylaminoethyl methacrylate, methoxyethyl acrylate, methoxyethyl methacrylate, glycidal methacrylate, and the like. Additional examples of appropriate acrylic adhesives are described in Satas, "Acrylic Adhesives," Handbook of pressure-Sensitive Adhesive Technology, 2nd ed., pp. 396-456 (D. Satas, ed.), Van Nostrand Reinhold, New York (1989). The acrylic adhesives are commercially available (National Starch and Chemical Corporation, Bridgewater, NJ; Solutia, MA). Further examples of polyacrylate-based adhesives are as follows, identified as product numbers, manufactured by National Starch (Product Bulletin, 2000, DURO-TAK® is a trademark of National Starch adhesives): 87-4098, 87-2287, 87-4287, 87-5216, 87-2051, 87-2052, 87-2054, 87-2196, 87-9259, 87-9261, 87-2979, 87-2510, 87-2353, 87-2100, 87-2852, 87-2074, 87-2258, 87-9085, 87-9301 and 87-5298. DURO-TAK® 87-2287 and 87-4287 both are polymeric adhesive derived from monomer compositions that are similar: 5.2 wt% 2-hydroxyethyl acrylate, about 20-40 wt% vinyl acetate, and about 55-75 wt% 2-ethylhexyl acrylate; and these two polymeric adhesives are provided solubilized in ethyl acetate in solids content of about 40-50 wt%. The DURO-TAK® 87-2287 adhesive is derived from a monomer composition of vinyl acetate, 28%; 2-ethylhexyl acrylate, 67%; hydroxyethyl acrylate, 4.9%; and glycidyl methacrylate, 0.1%, see U.S. Patent Number 5,693,335.

Acrylic polymers suitable for use in embodiments of the patch described herein can contain cross-linked or non-cross-linked polymers or both. The polymers are cross-linked by known methods to provide the desired polymers. In some embodiments, the adhesive is a polyacrylate adhesive having a glass transition temperature (T9) less than -10 °C, more preferably having a T9 of about -20 °C to about -30 °C. The molecular weight of the polyacrylate adhesive, expressed as weight average (MW), generally ranges from 25,000 to 10,000,000, such as from 50,000 to about 3,000,000 or, alternatively from 100,000 to 1,000,000 prior to any cross-linking reactions. Upon cross-linking the MW approaches infinity, as known to those involved in the art of polymer chemistry.

As discussed above, the reservoir (e.g., reservoir 3 of Fig. 1 or Fig. 2) of a patch as described herein may be formed using a polymeric composition, which may or may not be free of undissolved components. A reservoir as contemplated herein includes an amount of fentanyl base sufficient to induce and maintain analgesia in a human for about 1 day. The drug reservoir comprises 0.14 to 0.3 mg/cm$^2$ of fentanyl base. In certain embodiments, the base form of fentanyl is dissolved or dispersed. In other embodiments the fentanyl base is completely dissolved.

In particular embodiments, the material forming the reservoir of a patch according to the present description has a solubility for the drug of about 0.5 wt% to about 25 wt% of the total polymer composition; such as about 1 wt% to about 15 wt%; or alternatively about 2 wt% to about 12 wt% of the total polymer composition 4. The reservoir utilized in a patch as described herein, with or without the adhesive coating 6, may exhibit a thickness of about 0.005-0.25 mm (0.2-10 mils), such as 0.0125-0.0375 mm (0.5-1.5 mils), or alternatively about 0.0125 - 0.03 mm (0.5-1.25 mil), or about 0.02-0.03 mm (0.8-1.2 mil), such as about 0.023-0.028mm (0.9-1.1 mil). In some such embodiments, the material forming the reservoir 3 has a solubility for fentanyl of about 0.5 wt% to about 25 wt% of the total polymer composition; such as about 1 wt% to about 15 wt%; or alternatively about 2 wt% to about 12 wt%; or about 4 wt% to about 10 wt% of the total polymer composition. One way of determining solubility of the drug in the adhesive is by casting matrix drug reservoir layers of different drug concentrations on backing materials and storing the cast materials at room temperature over a period of time and determining the appearance of drug crystals. If crystals appear after 1 month of storage at a particular concentration, that concentration can be considered to be the solubility limit of the drug in the matrix adhesive. The presence of crystals or undissolved materials can be determined by microscopy or by X-ray diffraction analysis.

The reservoir 3 included in a patch as described herein may optionally contain additional components such as, additives, permeation enhancers, stabilizers, dyes, diluents, plasticizers, tackifying agents, pigments, carriers, inert fillers, antioxidants, gelling agents, anti-irritants, vasoconstrictors and other materials as are generally known to the transdermal art, provided that such materials are present below saturation concentration in the reservoir.

If desired, permeation enhancers can be useful for increasing the skin permeability of the drug or drugs to achieve delivery at therapeutically effective rates. Such permeation enhancers can be applied to the skin by pretreatment or concurrently with the drug, for example, by incorporation in the reservoir. A permeation enhancer should have the ability to enhance the permeability of the skin for one, or more drugs or other biologically active agents. A useful permeation enhancer would enhance permeability of the desired drug or biologically active agent at a rate adequate to achieve therapeutic plasma concentrations from a reasonably sized patch (e.g., about 2 to 25 cm$^2$, although it may be larger). Some useful permeation enhancers include non-ionic surfactants; one or more can be selected from the group including glyceryl mono-oleate, glyceryl mono-laurate, sorbitan mono-oleate, glyceryl tri-oleate, and isopropyl myristate. The non-ionic surfactant can be used in the amount of about 0.1 to 30 wt % solids to the total composition of the reservoir layer. Examples of permeation enhancers include, but are not limited to, fatty acid esters of alcohols, including glycerin, such as capric, caprylic, dodecyl, oleic acids; fatty acid esters of isosorbide, sucrose, polyethylene glycol; caproyl lactylic acid; laureth-2; laureth-2 acetate; laureth-2 benzoate; laureth-3 carboxylic acid; laureth-4; laureth-5 carboxylic acid; oleth-2; glyceryl pyroglutamate oleate; glyceryl oleate; N-lauroyl sarcosine; N-myristoyl sarcosine; N-octyl-2-pyrrolidone; lauraminopropionic acid; polypropylene glycol-4-laureth-2; polypropylene glycol-4-laureth-5dimethy- I lauramide; lauramide diethanolamine (DEA). Preferred enhancers include, but are not limited to, lauryl pyroglutamate (LP), glyceryl monolaurate (GML), glyceryl monocaprylate, glyceryl monocaprate, glyceryl monooleate (GMO), oleic acid, N-lauryl sarcosine, ethyl palmitate, laureth-2, laureth-4, and sorbitan monolaurate. Additional examples of suitable permeation enhancers are described, for example, in U.S. Pat. Nos.: 5,785,991; 5,843,468; 5,882,676; and 6,004,578. However, in certain embodiments, no permeation enhancer is used.

The reservoir may comprise diluent materials capable of reducing quick tack, increasing viscosity, and/or toughening the matrix structure, such as polybutylmethacrylate (ELVACITE, manufactured by ICI Acrylics, e.g., ELVACITE 1010, ELVACITE 1020, ELVACITE 20), high molecular weight acrylates, i.e., acrylates having an average molecular weight of at least 500,000, and the like. If used, such larger molecular weight acrylate materials, e.g., ELVACITE, are not polymerized into the adhesive polymers but rather are blended in. Thus, the monomers for polymerization of the adhesive do not include such ELVACITE type large molecular weight acrylates (or macromonomer acrylates).

In certain embodiments, a plasticizer or tackifying agent is incorporated in the adhesive composition to improve the adhesive characteristics. Examples of suitable tackifying agents include, but are not limited to, aliphatic hydrocarbons; aromatic hydrocarbons; hydrogenated esters; polyterpenes; hydrogenated wood resins; tackifying resins such as ESCOREZ, aliphatic hydrocarbon resins made from cationic polymerization of petrochemical feedstocks or the thermal polymerization and subsequent hydrogenation of petrochemical feedstocks, rosin ester tackifiers, and the like; mineral oil and combinations thereof.

If used, the tackifying agent employed should be compatible with the polymers or blend of polymers. For example, the styrenic block copolymers can be formulated with rubber compatible tackifying resins, end- block compatible resins such polymethyl styrene, or plasticizers such as mineral oil. In certain embodiments, a patch as described herein may include a reservoir formed using a polymer material, a tackifier and a mineral oil plasticizer, wherein the polymer is about 5-50% of the total adhesive composition, the tackifier is about 30-85% of the total adhesive composition, and the mineral oil is about 2-40% of total adhesive composition.

A patch as described herein may further comprise a peelable protective layer 5. The protective layer 5 may be made of, for example, a polymeric material that may be optionally metallized. Examples of the polymeric materials include polyurethane, polyvinyl acetate, polyvinylidene chloride, polypropylene, polycarbonate, polystyrene, polyethylene, polyethylene terephthalate, polybutylene terephthalate, paper, and the like, and a combination thereof. In some embodiments, the protective layer comprises a siliconized polyester sheet.

A wide variety of materials can be used for fabricating the various layers of the transdermal delivery patches described above. This disclosure therefore contemplates the use of materials, other than those specifically disclosed herein, including those which may hereafter become known to the art to be capable of performing the necessary functions.

In one embodiment, a patch as described herein has the structure shown in FIG. 1 , in which the patch has only three layers, i.e., a backing layer, the single phase reservoir matrix layer with totally dissolved fentanyl base, and a release liner. The reservoir matrix is formed using a polyacrylate adhesive and only one opioid agent, which is fentanyl base. In one such embodiment, there is no significant amount of permeation enhancers, tackifier, fillers, etc. In other such embodiments, where there are such materials, they are present only in de minimis amount and do not have substantive impact on drug delivery. The polyacrylate adhesive used in such embodiments, may consist of a single copolymeric chemical entity, such as a copolymer of monomers vinyl acetate, 2-ethylhexyl acrylate, and hydroxyethyl acrylate, e.g., the terpolymer DURO-TAK® 87-4287 adhesive or copolymer DURO-TAK® 87-2287 adhesive.

Administration of the Drug

On application to the skin, the drug in the drug reservoir 3 of the transdermal patch 1 diffuses into the skin where it is absorbed into the bloodstream to produce a systemic analgesic effect. The onset of analgesia depends on various factors, such as, potency of the drug, the solubility and diffusivity of the drug in the skin, thickness of the skin, concentration of the drug within the skin application site, concentration of the drug in the drug reservoir, and the like.

In one embodiment, to maintain continuous analgesia, after one day of deployment, the old (i.e., used) patch that has remained on the skin for a day is removed and a fresh patch is applied to the skin, preferably to a new location. For example, after the blood drug level has reached a therapeutic level, the patch would be sequentially removed and replaced with a fresh patch at the end of the administration period to provide relief from chronic pain. Preferably the old (i.e., used) and the new patch are not attached to the skin simultaneously, or the overlapping time period is insignificantly small (e.g., in minutes) for convenience of application and providing a more predictable analgesic result. At steady state, the absorption of the drug from the fresh patch into the new application area and the systemic circulation and the absorption of the residual drug within the previous patch application site occur at a rate that maintains blood levels of the opioid drug within an acceptable range.

To achieve therapeutic blood level more rapidly, the health care giver or the patient may choose to apply one patch or multiple patches initially and after one day (i.e., about 24 hours) maintain a smaller number of patches on the skin, e.g., leaving only one patch on the skin with replacement daily (i.e., about every 24 hours).

It has been found that a blood plasma concentration of fentanyl of between about 0.02 to about 10 ng/ml is typically therapeutically effective. Within this range, a blood plasma concentration of between about 0.3 to about 3 ng/ml may be targeted. Within such ranges, as usually is the case for most drugs, less fluctuation over time is desirable over more fluctuation because of a more consistent analgesic effect over time associated with a less fluctuation in drug concentration in the blood.

With a daily replacement program using once-a-day fentanyl patches according to the present invention, a much smaller fluctuation of drug concentration in the plasma may be achieved relative to multi-day patch regimens, such as, for example, 3-day patch and administration regimen. Thus, in one embodiment, a patch and method of administration as described herein provides a lower single dose $C_{max}$, a higher steady state $C_{min}$, and a smaller difference between $C_{max}$ and $C_{min}$ at steady state that is achieved by a multi-day patch and administration regimen for delivery of the same drug. In a specific embodiment, the present disclosure provides a patch and method of daily replacement that achieve a lower single dose $C_{max}$, a higher steady state $C_{min}$, and a smaller difference between $C_{max}$ and $C_{min}$ at steady state than provided by a 3-day patch and three-day or twice a week application regimen.

In particular embodiments, a patch as described herein is a 1-day patch providing a single dose profile of fentanyl base (i.e., a single patch is applied and then removed at the end of 24 hours without replacement) wherein the $C_{max}$ ranges from about 0.45 to about 5.5 ng/ml, such as from about 0.9 to about 2.7 ng/ml, or alternatively from about 1.2 to about 2.5 ng/ml after the one day application with a nominal delivery rate of 100 μg/h. For dose strength different from a nominal delivery rate of 100 μg/h, the $C_{max}$ ranges are scaled accordingly by differences in area. In one such embodiment, the drug is fentanyl. In certain such embodiments, the base form of fentanyl is dissolved or dispersed. In other embodiments the fentanyl base is completely dissolved.

In a specific embodiment, a patch as described herein is a transdermal fentanyl patch of 100μg/h dose strength exhibiting a steady state $C_{max}$ ranging from about 0.7 to about 12 ng/ml, such as about 1.5 to about 6 ng/ml, or alternatively about 2 to about 5.5 ng/ml, and furthermore, from about 3 to about 5 ng/ml. Such a fentanyl patch may additionally exhibit a steady state $C_{min}$ ranging from about 0.5 to about 10 ng/ml, or from about 1 to about 5.5 ng/ml, or alternatively, from about 1 to about 4 ng/ml, or from about 1 to about 3 ng/ml. For patches of lower or higher dose strengths, the steady state $C_{max}$ ranges are scaled accordingly, e.g., based on the difference in area. The fentanyl present in such embodiments is the base form of fentanyl and is dissolved or dispersed. In other such embodiments the fentanyl base is completely dissolved.

A patch as disclosed herein may also provide a targeted difference between $C_{max}$ and $C_{min}$ for a given dose strength. For example, in one embodiment of a patch as described herein that provides a dose strength of 100 μg/h, the daily swing between $C_{max}$ and $C_{min}$ ranges from about 0.025 to about 13.0 ng/ml, such as about 0.25 to about 2 ng/ml, or from about 0.3 to about 1.6 ng/ml, or alternatively, from about 0.4 to about 0.8 ng/ml. certain such embodiments, the base form of fentanyl is dissolved or dispersed. In other embodiments the fentanyl base is completely dissolved.

Additionally a patch as disclosed herein, designed to provide a steady state for a dose strength of 100 μg/h, may also provide a drug fluctuation, defined as $(C_{max} - C_{min})/C_{avg}$, that may be less than 100%, such as less than 90%, or alternatively less than 80%, or furthermore less than 70%, or from about 30% to 65%. In certain such embodiments, the base form of fentanyl is dissolved or dispersed. In other embodiments the fentanyl base is completely dissolved.

For devices of dose strengths that are different from 100 $\mu$g/h, the daily swing between $C_{max}$ and $C_{min}$ ranges can be scaled accordingly. The daily swing between normalized $C_{max}$ and $C_{min}$ ranges from about 2 to about 100 ng/ml(mg/h), such as from about 4 to about 50 ng/ml(mg/h), or alternatively, from about 5 to about 20 ng/ml(mg/h), or from about 5 to about 15 ng/ml(mg/h). For devices of dose strength that is different from 100 $\mu$g/h, the daily swing between normalized $C_{max}$ and normalized $C_{min}$ ranges can be scaled accordingly (such as for the 25$\mu$g/h, 50$\mu$g/h, 75$\mu$g/h, and 12.5$\mu$g/h dose strengths). To be bioequivalent to a DURAGESIC® DTRANS® fentanyl transdermal system, the 90% confidence interval of the steady state $C_{max}$ ratio of a new transdermal system to that of the DURAGESIC® DTRANS® system should be within 80% to 125%. Also, the 90% confidence interval of the AUCss ratio of a new transdermal system to that of a DURAGESIC® DTRANS® system of the same dose strength should be within 80% to 125%. Thus, to test for bioequivalency, new transdermal systems for delivery of fentanyl are to be tested against DURAGESIC® DTRANS® systems to show that the 90% confidence interval of the above-mentioned pharmacokinetic parameter ratios of the new system to that of the DURAGESIC® DTRANS® system are within 80% to 125%.

With a nominal delivery rate of 100 $\mu$g/h, the $AUC_{inf}$ (i.e., the AUC to a time of infinity after a single dose) of a single dose application of a once-a-day fentanyl patch as described herein may range from about 15 to about 200 ng-h/ml, such as from about 30 to about 140 ng-h/ml after the daily application of a transdermal fentanyl patch with a nominal delivery rate of 100 $\mu$g/h. For a transdermal fentanyl product, $AUC_{SS}$ and $AUC_{inf}$ have been demonstrated to be bioequivalent (see Sathyan, et al "Evaluation of the bioequivalence of two transdermal fentanyl systems following single and repeat applications" Current Medical Research and Opinion 21(12) 1961-1968, 2005). For patches of lower or higher dose strengths, the steady state $AUC_{inf}$ ranges are scaled proportional to the patch area (or size). In certain such embodiments, the base form of fentanyl is dissolved or dispersed. In other embodiments the fentanyl base is completely dissolved.

A fentanyl patch may exhibit a normalized steady state $C_{max}$ for the drug delivered ranging from about 7 to about 120 ng/ml(mg/h), such as from about 15 to about 60 ng/ml(mg/h), or alternatively, from about 20 to about 55 ng/ml(mg/h), or from about 30 to about 50 ng/ml(mg/h). On administration over skin a patch as described herein may provide a steady state drug flux of about 0.1 to about 20 $\mu$g/(cm$^2$h); such as about 0.75 to about 10 $\mu$g/(cm$^2$h); or from about 1 to about 8 $\mu$g/(cm$^2$h); or alternatively from about 1.5 to about 5 $\mu$g/(cm$^2$h); or from about 2 to about 3 $\mu$g/(cm$^2$h). Steady-state administration rates obtainable according to this disclosure range from about 0.1 to about 500 $\mu$g/h; such as from about 1 to about 300 $\mu$g/h; or alternatively, from about 2 to about 250 $\mu$g/h; or from about 5 to about 200 $\mu$g/h. Nominal steady-state administration can range from, e.g., 12.5 $\mu$g/h, 25 $\mu$g/h, 50 $\mu$g/h, 75 $\mu$g/h, 100 $\mu$g/h, and 125 $\mu$g/h dose strengths. In certain such patches, the base form of fentanyl is dissolved or dispersed. In other patches the fentanyl base is completely dissolved.

A transdermal fentanyl patch may exhibit a normalized steady state $C_{min}$ ranging from about 5 to about 100 ng/ml(mg/h), such as about 10 to about 55 ng/ml(mg/h), or alternatively, from about 10 to about 40 ng/ml(mg/h), or from about 10 to about 30 ng/ml(mg/h). The transdermal patch is about 1 to about 100 cm$^2$, such as about 1 to about 40 cm$^2$; or from about 5 to about 38 cm$^2$; or alternatively, from about 10 to about 35 cm$^2$, or from about 10 to about 35 cm$^2$. On administration over skin, in terms of the amount of fentanyl present in the patch and flux, the transdermal fentanyl patch may generally exhibit a steady state drug flux of about 0.1 to 20 $\mu$g/(cm$^2$h); such as about 1 to about 10 $\mu$g/(cm$^2$h); about 1.5 to about 8 $\mu$g/(cm$^2$h); about 2.8 to about 5 $\mu$g/(cm$^2$h); or from about 3 to about 3.6 $\mu$g/(cm$^2$h). In certain such patches, the base form of fentanyl is dissolved or dispersed. In other patches the fentanyl base is completely dissolved.

A patch may be a 1-day fentanyl patch the delivers fentanyl in a manner that approximates, for example, in a manner that is bioequivalent to, the 3-day DURAGESIC® DTRANS® system. In one such embodiment, both the surface area and the thickness of the 1-day patch are reduced relative to the 3-day patch. For example, the 1-day patch may have a skin-contacting area about 0.5 to 0.85, such as about 0.6 to 0.8, or alternatively, 0.7 to 0.77, or from about 0.71 to 0.76 that of the 3-day patch. Additionally, the thickness of the 1-day fentanyl reservoir can be about 0.25 to 0.75, such as about 0.4 to 0.6, or alternatively about 0.5 that of the reservoir included in the 3-day patch. In each such patch, the patch may include a polyacrylate fentanyl matrix.

Although a 1-day patch should deliver about 1/3 the amount of fentanyl delivered by a three day patch, we have discovered that the 1-day patch may have a skin-contacting area of larger than 1/3 that of the 3-day patch of the same dose strength, thus between 1/3 to 0.9 that of the 3-day patch. To compare the surface areas of two patches, consider the normalized area of a patch as defined by dividing the patch drug reservoir area by the dose strength and dividing by the number of days the patch is to be used to deliver the drug (i.e., for the 3-day patch, the normalized area is obtained by dividing 42 cm$^2$ by 100 $\mu$g/h by 3=0.14 cm$^2$ per $\mu$g/h per day= 5.8 cm$^2$ per mg of nominal delivery amount). In comparison, the 1-day patch has a normalized area larger than 0.14 cm$^2$ per $\mu$g/h per day (i.e., larger than 5.8 cm$^2$ per mg of nominal delivery amount), which is that of the 3-day patch. This is true even when the thickness of drug reservoir (e.g., a drug containing matrix) of the 1-day patch is less than that of the 3-day matrix patch.

In terms of normalized area based on nominal dose strength, i.e., body-contacting surface area divided by the nominal dose strength (e.g., the normalized surface of a 100 $\mu$g/h delivery rate dose strength patch having a surface area of 42 cm$^2$ is 0.42 cm$^2$ per $\mu$g/h), for the delivery of 1-day worth of fentanyl, calculated according to the nominal dose strength, embodiments of a 1-day patch as disclosed herein may have a normalized area of about 0.2 to 0.4 cm$^2$ per μg/h per day (i.e., 8.5 to 16.5 cm$^2$ per mg), such as about 0.25 to 0.36 cm$^2$ per μg/h per day (i.e., 10.5 to 15 cm$^2$ per mg), or alternatively, about 0.28 to 0.32 cm$^2$ per μg/h per day (i.e., 11.5 to 13.5 cm$^2$ per mg).

the fentanyl base loading per surface area in a 1-day fentanyl patch according to the present invention is less than that of a 3-day fentanyl matrix patch, i.e., is 0.14 to 0.3 mg/ cm$^2$, as the DURAGESIC® DTRANS® fentanyl transdermal system has about 0.4 mg/cm$^2$. However, we have found that for bioequivalence to DURAGESIC® DTRANS® fentanyl transdermal system, the loading in a 1-day patch is more than just 1/3 that of the 3-day patch, i.e., more than 0.13 mg/cm$^2$. Therefore, the fentanyl base loading per surface area is 0.14 to 0.3 mg/cm$^2$, such as about 0.16 to 0.25 mg/cm$^2$, or alternatively, about 0.18 to 0.22 mg/cm$^2$.

The loading of a smaller amount of drug in a patch and reducing the residual drug in a used patch may offer advantages in reducing or deterring drug abuse. In an embodiment of a 1-day fentanyl base patch according to the present invention the fentanyl base content per patch may be 4 to 8 mg, such as 5 to 7 mg, or alternatively, 5.5 to 6.5 mg for a patch of 100μg/h nominal dose strength. In terms of normalized value calculated for scaling to different dose strengths such as 25 μg/h, 50 μg/h, 125 μg/h, etc., the normalized fentanyl content, (which is the fentanyl content divided by the dose strength), is about 0.04 to 0.08 mg.h/μg, such as 0.05 to 0.07 mg.h/μg, or alternatively 0.055 to 0.065 mg.h/μg. It was found that 1-day patches exhibiting such loading provided therapeutic analgesia similar to the 3-day DUROGESIC DTRANS®. For comparison, the 3-day DUROGESIC DTRANS® 100μg/h patches include about 17mg of fentanyl, which is about 0.17 mg.h/μg.

A patch as described herein may be designed to provide a desired utilization of drug (%-utilization). For example, the patch described herein may be a 1-day patch providing a drug utilization of larger than about 30%, such as at least 35%, or alternatively at least 40%, or 40% to 50%. It may be a fentanyl patch providing a utilization fentanyl larger than about 30%, such as at least 35%, or alternatively at least 40%, or 40% to 50%. In certain such patches, the base form of fentanyl is dissolved or dispersed. In other patches the fentanyl base is completely dissolved.

Administration of a patch is maintained for no greater than one day. The method of administration as disclosed herein calls for daily replacement (one-day use). Again, without being bound by or limited to a particular theory, it is thought that in light of the depot effect, if only one patch is applied on the first day and replaced daily, the blood plasma drug level will gradually increase and may take 2 or more

days to come to a steady state for a therapeutic effect. In another aspect, the present disclosure provides a method of administering fentanyl base by applying two or more patches initially on day one and subsequently daily applying a new patch to replace a day-old patch starting from day two, such that the drug plasma level is within a therapeutic range in a shorter period of time, e.g., in about one day. Some of the two or more patches can be removed on days following the first day such that at steady state, only one patch remains on the skin for analgesia.

Thus, where fentanyl is to be delivered to a patient for analgesia, and the patient may require a relatively higher plasma concentration of fentanyl, such, for example, an opioid tolerant patient, in order to achieve therapeutic effect, large doses of fentanyl, such as two, three or more patches can be administered to the patient on the first day in order to ensure a therapeutically effective plasma level of fentanyl is achieved on the first day. On day two, one or more of the patches can be replaced, and on day three, all of the patches can be removed, with only one new patch being reapplied. Variations of the above procedure can be used, such as replacing only one patch after day one and/or removing a different number of used patches. The use of multiple patches initially is particularly suitable for patients who are not naive to the drug but already have a level of tolerance. Using two or more patches initially and gradually reducing the number of patches on the skin can quickly bring the blood drug level to the steady state level. To determine whether a patient is naive to the drug, the medical record can be referenced in the process.

In another aspect, a patient can be determined (e.g., on the basis of the medical record) to be naive to the opioid drug, e.g., such as fentanyl, and that the patient will benefit from a blood drug level lower during the first day than the steady state blood level. Based on this determination, only one transdermal opioid patch is applied and replacement is done daily (every 24 hours) so that only one patch is used for delivery of the drug at a time. In certain cases it may be determined to be desirable to achieve the steady state target plasma drug concentration in 2 to 3 days, for example, for a fentanyl naive patient. The regimen of starting initially by applying only one patch on day one will allow such gradual increase to be carried out and yet achieve acceptable analgesia due to the drug-naiveness of the patient.

The transdermal devices disclosed can be included in a kit that contains the device and includes an instruction print, such as an insert or printings on a container, and the like that provides instruction on the how long each patch is to be applied to a patient and how often the patch is to be removed and replaced with a new patch. The instruction of use can include a regimen of patch application as described above, including instruction such as applying one patch initially and replacing daily, or applying two patches initially and removing both but replacing only one after one day, or other alternative regimens. The instruction of use can also include a brief description of the drug, the backing, the reservoir, the adhesive, pharmacokinetic information, information on disposing a control substance (e.g., fentanyl) and warnings.

Methods of Manufacture

The transdermal devices are manufactured according to known methodology, such as those described in U.S. Patent Publication No. 2003002682. In brief, a transdermal drug delivery device can be made by forming a matrix drug-containing adhesive reservoir layer on a backing material, laying a protective film on the matrix reservoir and cutting to form a device of the desired size. The drug-containing matrix can be formed on the backing by casting a drug/adhesive solution on the backing layer and allowing solvent to evaporate and escape from the drug/adhesive, thereby forming the drug-containing matrix with a desired thickness and drug concentration.

Simulation

Below are examples of specific embodiments for of the once-a-day fentanyl patch:

Simulation 1: Steady State, 1-day Patch vs. 3-day Patches

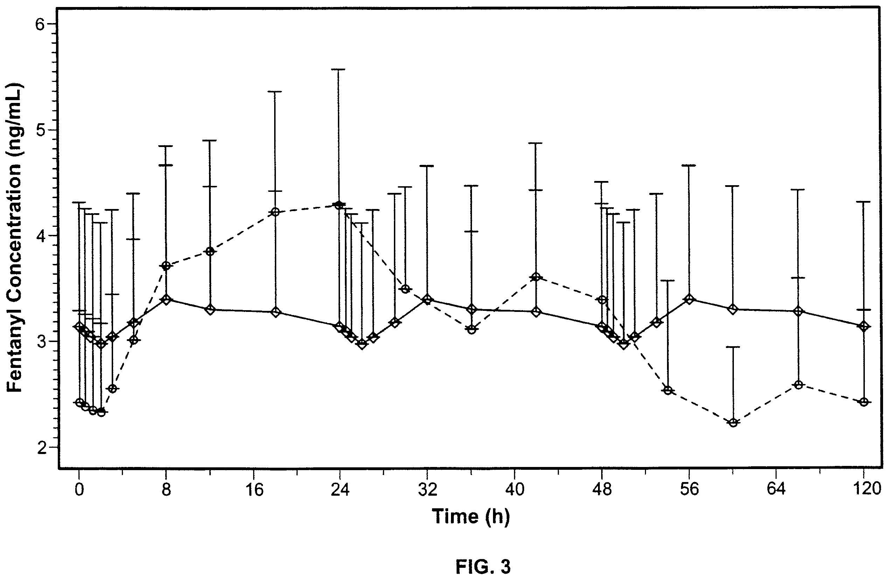

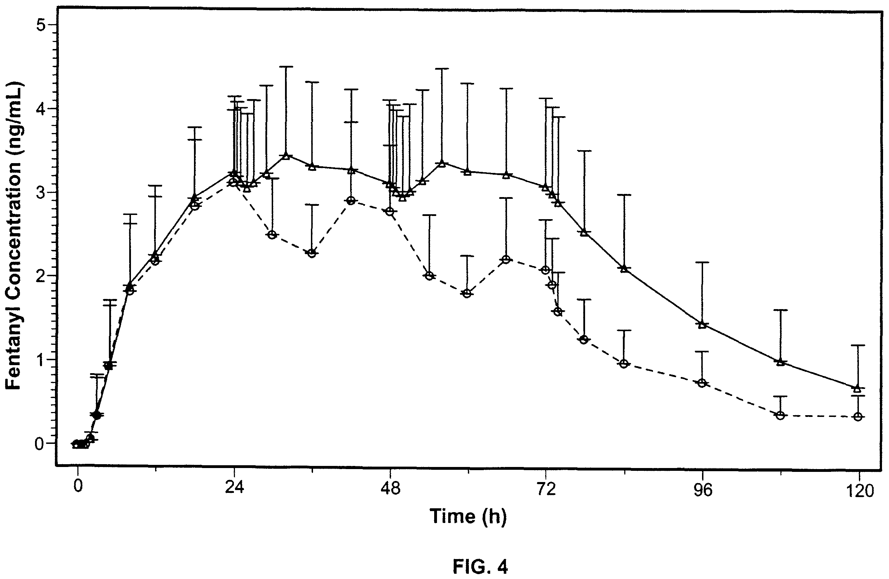

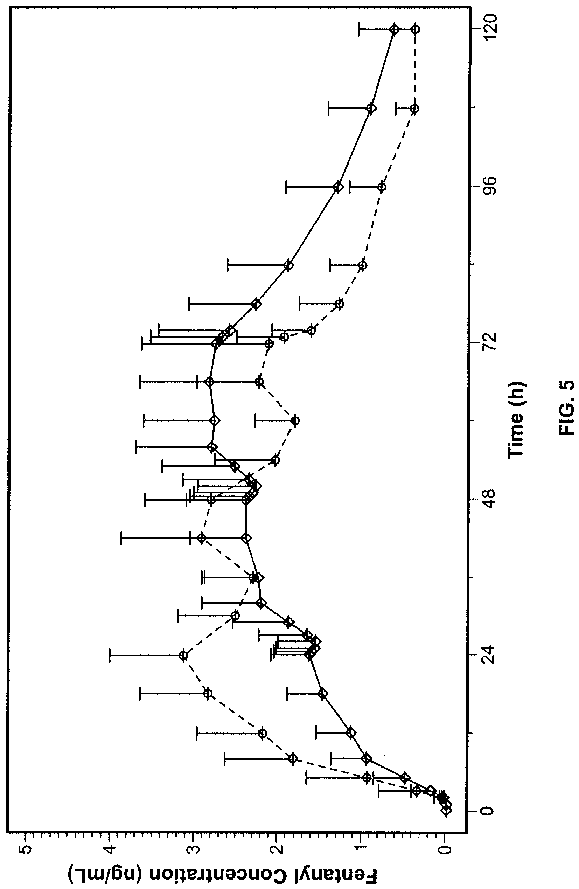

FIG. 3 shows a simulation graph of the serum fentanyl level at steady state comparing the use of 1-day transdermal fentanyl base patches versus using 3-day transdermal base patches (DUROGESIC® DTRANS® matrix patches, available in the United Kingdom from Janssen-Cilag). The actual data of 3-day patches applied to the skin for three days before removal without replacement were used as the basis for the continuous effect of steady state administration. The actual data for the 3-day patches are the same as those shown in FIG. 4 and FIG. 5.

The actual individual serum fentanyl concentration data after a 3-day administration of a single Transdermal DTRANS® fentanyl 100 $\mu$g/h matrix system were used for the purpose of simulation. For the single administration of a transdermal fentanyl system, the serum fentanyl concentration at time t, C(t), during the wearing of a system is based on the data collected. The serum fentanyl concentration post-removal of transdermal fentanyl system is approximated by a first-order decay function using the elimination rate constant, k, for each individual and this constant is assumed to be the same when the system is removed after 24 hours of use. The serum fentanyl concentration at hour t, after the application of the second system, $C(t)^{2nd}$ and the simultaneous removal of the first system is simulated according to the following equation based on superposition principle:

$$C(t)^{2nd} = C(t) + C(\tau)e^{-kt}$$

where $\tau$ is the dosing interval, e.g., 24 hours if the first system is removed and the second system is applied at the end of 24 hours, C(t) is the serum concentration observed at t hours after the application of a single system, $C(\tau)$ is the serum concentration at $\tau$ hour after the application of a single system, and k is the apparent first order decay rate constant describing the serum fentanyl concentration post-system removal at hour $\tau$. The serum fentanyl concentration after the application of Nth system is simulated according to the following equation. $C(\tau)e^{-kt}$ is the decaying concentration due to the old patch applied at $\tau$; $\tau$ is the dosing interval, 24 or 72 hours, etc., while t is related to the elapsed time from the last patch application.

$$C(t)^{N-th} = C(t) + C(\tau)e^{-kt}\frac{1-e^{-k(N-1)\tau}}{1-e^{-k\tau}}$$

At steady state (when N approaches infinity), the plasma fentanyl concentration is simulated according to the following equation:

$$C(t)^{SS} = C(t) + C(\tau)e^{-kt}\frac{1}{1-e^{-k\tau}}$$

The serum fentanyl concentration data after a single application of the 3-day acrylate matrix fentanyl patch for 37 healthy subjects were used as the basis for simulation of the 3-day system and the 1-day system. Patches that were approximately half (52% of) the size of the DUROGESIC® DTRANS® matrix patches 100 $\mu$g/h, and having the same composition were used as the 1-day patches for the simulation. Thus, the scaling factor for obtaining the parameters for the 1-day system of the same material construction (except in surface area) by simulation based on data from a 3-day patch is 0.52. The value for 52% was arrived at by dividing the $C_{avg}$ (average concentration) of a 3-day acrylate matrix fentanyl patch (DUROGESIC® DTRANS® matrix patch) applied for only one day by the $C_{avg}$ of a 3-day acrylate matrix fentanyl patch applied for three days. Since a 3-day patch works for three days, its delivery for the first day is assumed to be an amount, which if repeated three times, will be equivalent to the three day patch delivering for three days. The DUROGESIC® DTRANS® matrix patch (dose strength 100 µg/h) was used as the 3-day patches. Therefore the 1-day patches have the same matrix thickness and drug concentration as the DUROGESIC® DTRANS® matrix patch but were 21cm$^2$ each rather than 42 cm$^2$. In this simulation, the 3-day patches were applied with replacement once every three days (72 hours) with one patch on the skin at any given time. The 1-day patches were simulated as applied with replacement once every day (24 hours) with one patch on the skin at any given time.

The graph shows the excerpt of a mathematically simulated steady state (as time approaches infinity) 72-hour section. For convenience, in the graph of FIG. 3, the hours are shown to span a period of 72 hours at steady state starting at zero hour (i.e., the curves are continuous, repeating before the zero hour and after the 72 hour). In FIG. 3, the dash line (----) curve with the open circle data points represents simulated serum fentanyl concentration values for the 3-day patches, the solid line curve with the diamond data points represents simulated values for the 1-day patches. In this simulation, although not included in the excerpt that is depicted in the graph of FIG. 3, the 1-day patches took about 3 days to come to essentially a steady state condition. In the first two days (before steady state was achieved) when the concentration was rising, the concentration after 24 hours was only about half that of the steady state $C_{max}$ and the concentration after 48 hours was only about 70% that of the steady state $C_{max}$ similar to what is shown in FIG. 5 for the application of 1-day patches. The 3-day 100 µg/h patches were replaced once every three days. It is noted that for the 100 µg/h patches, the mean $C_{max}$ was about 4.7 ng/ml and the mean $C_{min}$ was about 2.1 ng/ml. For the 1-day patches, the mean $C_{max}$ was about 3.5 ng/ml and the mean $C_{min}$ was about 2.9 ng/ml. Thus, the 1-day patches had a $C_{max}$ that was lower than the $C_{max}$ of the 3-day patches. However, the 1-day patches had a $C_{min}$ that was higher than the $C_{min}$ of the 3-day patches, resulting in a smaller swing between the $C_{max}$ and $C_{min}$ for the 1-day patches.

Simulation 2: 3-day application of 1-day patches. Double initial dose

FIG. 4 shows a simulation graph of the blood fentanyl level at the beginning of use of a transdermal fentanyl product comparing the use of 1-day transdermal fentanyl base patches versus actual data using 3-day transdermal base patches (DUROGESIC® DTRANS® matrix patches) one time without replacement. The same skin and patch parameters as SIMULATION 1 were used.

Patches that were half the size of the DUROGESIC® DTRANS® matrix patches and having the same composition were used as the 1-day patches for the simulation. The DUROGESIC® DTRANS® matrix patches were 100 µg/h patches of 42 cm$^2$ each. Therefore, the 1-day patches had the same matrix thickness and drug concentration as the DUROGESIC® DTRANS® matrix patch but were approximately 21 cm$^2$ each. For the 3-day patches, in an actual experiment, only one DUROGESIC® DTRANS® matrix patch was initially applied at zero hour and remained in place until removal after 72 hours. The 1-day patch was replaced every 24 hours. In the simulation, two 1-day patches were applied at zero hour. At 24 hours the two initial patches were removed and replaced with only one new 1-day patch. Subsequently the 1-day patch was replaced daily with a fresh patch. The final used patch was removed at 72 hours for both cases. The simulation generated data points started at the physical 24$^{th}$ hour data point of the 3-day patch because in both cases the same patch material and amount were used during the first 24 hours. In the graph of FIG. 4, the dash line (----) curve with the open circle data points represents data for the 3-day patches, the solid line curve with the triangle data points is the curve for the 1-day patches.

The actual experimental data show the daily swing of the 3-day patch with a gradual overall decline in fentanyl concentration in the blood. The simulated data show that when using patches half the size of DUROGESIC® DTRANS® matrix patches the blood fentanyl concentration can be maintained in a reasonably stable therapeutic level starting from the 24$^{th}$ hour. In fact, the daily fluctuation is much smaller than that of the 3-day patch.

Simulation 3: 3-day application of 1-day patches. Single initial dose

FIG. 5 shows a simulation graph of the serum fentanyl level at the beginning of a transdermal fentanyl product comparing the use of 1-day transdermal fentanyl base patches versus actual data using 3-day transdermal base patches (DUROGESIC® DTRANS® matrix patches). The same actual data for the 3-day patches as FIG. 4 were used. The same pharmacokinetic parameters as SIMULATION 1 were used.

Patches that were half the size of the DUROGESIC® DTRANS® matrix patches and having the same composition were used as the 1-day patches for the simulation. The DUROGESIC® DTRANS® matrix patches were 100 µg/h patches of 42cm$^2$ each, Therefore the 1-day patches had the same matrix thickness and drug concentration as the DUROGESIC® DTRANS® matrix patch but were approximately 21cm$^2$ each. The same actual physical experimental data from SIMULATION 2 were used for the 3-day patches. In the simulation, only one 1-day patch was applied at zero hour. At (i.e., after) 24 hours the initial patch was removed and replaced with one new 1-day patch. Subsequently the 1-day patch was replaced daily with a fresh patch. The final used patch was removed at 72 hours for both cases. The simulation generated data points started at zero hour. In the graph of FIG. 5, the dash line (----) curve with the open circle data points represents data for the 3-day patches, the solid line curve with the diamond data points represents simulated data for the 1-day patches. The simulated data show that using patches half the size of DUROGESIC® DTRANS® matrix patches only one at a time the blood fentanyl concentration will gradually increase to arrive at a level that is at the therapeutic level after 3 days. The 1-day patches took about 3 days to come to essentially a peak condition similar to the steady state condition of Simulation 1 using 1-day patches. In the first two days when the concentration was rising, the concentration after 24 hours was only about half that of the peak $C_{max}$, and the concentration after 48 hours was only about 75% that of the peak $C_{max}$. The dip in blood fentanyl level at the time of replacement was quite small as the blood fentanyl concentration continued on its upward swing to reach the steady-state level.

Experimental

Examples of specific embodiments are included below. The examples are offered for illustrative purposes only, and are not intended to limit the scope of the present disclosure in any way. Although a reservoir saturated with drug can be used, the adhesive-reservoir patches wherein the reservoir comprises a single phase polymeric composition free of undissolved components containing an amount of fentanyl at or below subsaturation concentration are disclosed in the following examples. In the following examples all percentages are by weight unless noted otherwise. It is noted that for the following examples, instead of making the 3-day patches for the experiments, one can also obtain DUROGESIC® DTRANS or DUROGESIC® SMAT (which is equivalent in structure to DUROGESIC® DTRANS) patches from vendors and cut them to right surface area as required for the experiments.

Example 1: 3-day Patch, 1 Time Application for 3 Days

Monolithic transdermal patches according to Fig. 1 that had the same construction as the commercial DUROGESIC® DTRANS® patches comprising about 8 wt% of fentanyl base were made and used. The matrix adhesive was National Starch DURO-TAK® 87-4287 polyacrylate adhesive. The resultant matrix was about 2 mils (0.05 mm) thick. The patches were made to have sizes of about 5.5 cm², 11 cm², 21 cm², 32 cm², and 42 cm² corresponding to nominal fentanyl dose strength delivery rates of 12.5 μg/h, 25 μg/h, 50 μg/h, 75 μg/h, and 100 μg/h. In the process of making the patches, a polyacrylate adhesive solution was prepared (98.35 kg National Starch 87-4287 of about 39wt% solids was diluted with 18.3 kg ethyl acetate). Then 3.35 kg of fentanyl base was added to the polyacrylate adhesive solution in amounts sufficient to generate a mixture containing 2.8 wt% of fentanyl in the adhesive solution and stirred to dissolve the drug. The solution was cast and the solvent was evaporated to result in a 2 mil (0.05mm) thick reservoir layer. A 1.7 mil (0.04mm) thick backing layer comprised of a multilaminate of polyethylene/polyurethane/polyester layer was laminated on to the adhesive drug reservoir layer using standard procedures. Individual patches were die-cut from this laminate in 11 cm², 21 cm², 32 cm², and 42 cm² sizes comprising respectively, 4.4, 8.5, 13, and 17 mg each of fentanyl, to generate monolithic transdermal patches containing about 0.4 mg/cm² of fentanyl base.

Subjects were randomly assigned to a treatment sequence and to wear a study system on the upper outer arm, upper chest, or upper back as per a randomization list. There were four treatment periods. Subjects selected were not dependent on opioids. During each treatment, the study system was worn for 72 hours on a new application skin site on the application area to which subjects were randomized (upper outer arm, upper chest, or upper back). For each subject the same area of the body was used throughout the study. There was a minimum washout period of at least 6 days and not more than 14 days between treatments. The washout period was to commence upon removal of the study system.

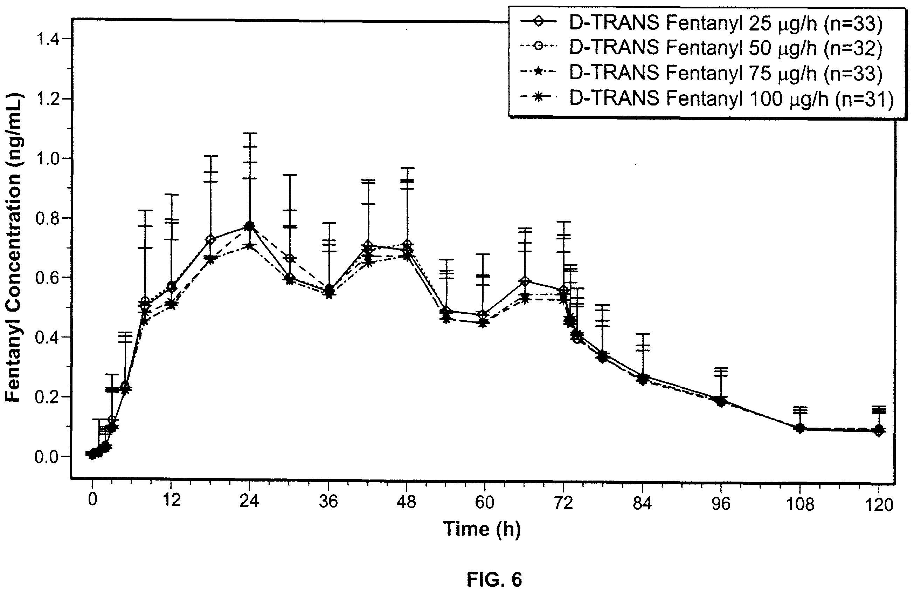

FIG. 6 shows the summary of the averaged data on the fentanyl concentration in the blood of the subjects in a normalized form (i.e., the data were normalized to the 25 μg/h dose rate (i.e., dose strength) by dividing the fentanyl concentration with the ratio factor of the nominal dose strength delivery rate by the 25 μg/h nominal dose strength delivery rate). Thus, the data for the 100 μg/h dose rate were divided by 4 to be compared to the curve for the 25 μg/h, which was original). In FIG. 6, the solid curve with diamond data points represents the data for the 25 μg/h dose rate. The dash curve with the open circle data points represents the data for the 50 μg/h dose rate. The dotted curve with the star data points represents the data for the 75 μg/h dose rate. The dash/dot (- - - -) curve with the asterisk data points represents the data for the 100 μg/h dose strength. FIG. 6 shows that different dose rates have about the same proportional changes with time in the fentanyl concentration. In a dimensionless, normalized form, the curves for each dose rate were about the same (within experimental errors, without significant statistical difference). Thus, in another normalized form in which the blood fentanyl concentration was divided by the $C_{avg}$, wherein $C_{avg}$ is the average plasma fentanyl concentration, the curves for the different dose strengths (not shown in graphs here) would be almost the same. For example, $C_{max}/C_{avg}$ and $C_{min}/C_{avg}$ within the first 48 hours would be about the same for each dose rate.

Example 2: 3-day Patch. Replaced Daily for 3 Days

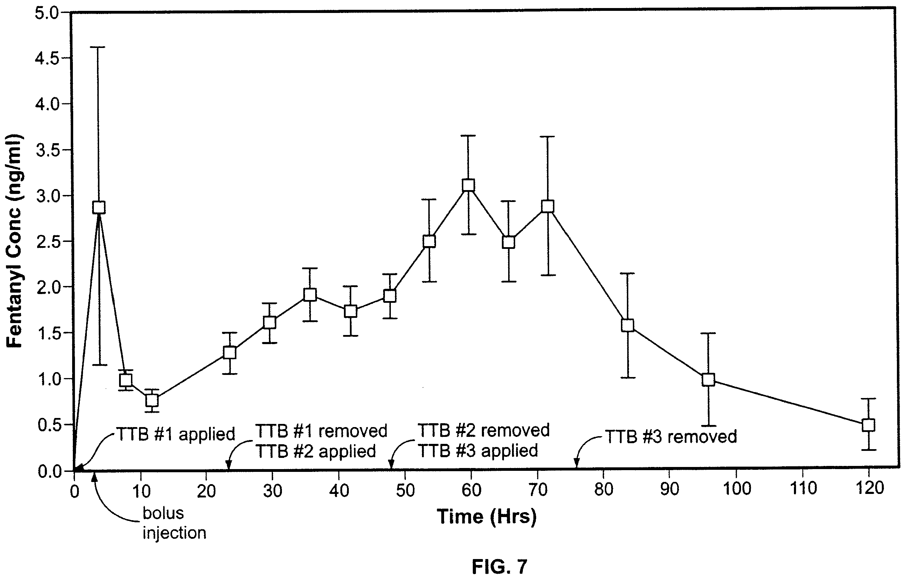

FIG. 7 represents actual serum fentanyl concentrations collected over time in using 3-day patches (that had the same construction as the commercial DURAGESIC® reservoir patches containing about 8 wt% of fentanyl base) when they were replaced once every 24 hours. Since the DUROGESIC® DTRANS matrix patches were made to be bioequivalent to the DURAGESIC® reservoir patches, using either kind of these patches would result in similar serum fentanyl concentration. Patches with dose strength of 75 $\mu$g/h were used. In this study (in which 11 colon-rectal surgery patients were tested), a first 75 $\mu$g/h patch was applied at the beginning at zero hour (approximately 2 hours prior to the induction of anesthesia). General anesthesia was induced with 2-4 mg/kg thiopental and a 3-5 $\mu$g/kg bolus of fentanyl up to a maximum dose of 400 $\mu$g. Thereafter, the patch was replaced every 24 hours. At (after) 72 hour, the patch (i.e. the third patch) was removed.

The data show a step-wise increase in serum concentration with each new patch administration. This result was not expected because it was assumed that the multi-day patches would deliver drug with kinetics consistent with the constant intravenous infusion of fentanyl. Because only one patch is applied to the skin at any given time, the expectation (similar to the case of constant infusion model) was that the serum fentanyl levels would be similar to that of the case as a single three day patch applied on the skin for three days, in which case serum fentanyl concentrations would rise to and maintain at approximately the steady-state concentration obtained after 24 hours. However, an accumulation effect with daily replacement resulting in gradual (or stepwise) increase of serum drug level over many days was found in delivery of opioid narcotic, such as fentanyl.

Example 3: 1-day Patch (with 87-2287 Adhesive), Replaced Daily for 3 or More Days

Monolithic transdermal patches according to Fig. 1 are prepared in 5, 11, 21, 31 and 42 cm$^2$ sizes comprising about 7 wt% of fentanyl base to correspond to 25 $\mu$g/h, 50 $\mu$g/h, 100 $\mu$g/h, 150 $\mu$g/h, and 200 $\mu$g/h (based on 0.52 scaling factor).

The matrix adhesive was National Starch DURO-TAK® 87-2287 polyacrylate adhesive. The DURO-TAK® 87-2287 adhesive polymer is an adhesive polymerized from vinyl acetate, 28%; 2-ethylhexyl acrylate, 67%; hydroxyethyl acrylate, 4.9%; and glycidal methacrylate, 0.1% (see USPN 5,693,335). An about 43wt% polyacrylate adhesive solution (National Starch 87-2287 in ethyl acetate) was prepared. Fentanyl base was added to the polyacrylate adhesive solution in amounts sufficient to generate a mixture containing about 3.4wt% of fentanyl in the adhesive solution and stirred to dissolve the drug. The solution was cast into a reservoir layer and the solvent is evaporated to result in a matrix layer of 1 mil (0.025mm) thickness. After solvent evaporation, a 3 mil thick backing layer comprised of a multilaminate of nonlinear LDPE layer/linear LDPE layer/nonlinear LDPE layer is laminated on to the adhesive drug reservoir layer using standard procedures. Individual patches are die-cut from this laminate in 5, 11, 21, 31 and 42 cm$^2$ sizes to have about 7 wt% each of fentanyl, to generate monolithic transdermal patches containing about 0.2 mg/cm$^2$ of fentanyl base.

Such patches are to be applied to an opioid-naive patient with the application of only one patch initially and replacement every 24 hours. The result is expected to be that the 1-day patches take about 3 days to come to essentially a steady state condition. The concentration after 24 hours is only about 50% to 60% that of the steady state $C_{max}$, and the concentration after 48 hours is only about 70% to 80% that of the steady state $C_{max}$. The result of the first three days is expected to be similar to that shown in FIG. 5 and similar to that of FIG. 3 at near steady state, after about 3 days, based on simulation. The values can be scaled according to the dose strength based on the size differences and that the normalized curves according to dose strength will substantially superimpose.

In another test, such patches are to be applied to a person who has substantially no such drug in the blood, by applying two patches initially at zero hour. Both initial patches are removed at the end of 24 hours and replaced with only one 1-day patch, which is thereafter replaced every 24 hours. The result is expected to be that the 1-day patches take about 1 day to come to the level of essentially steady state condition. The fentanyl concentration $C_{max}$ during the first 24 hours is about 3 ng/ml. The result of the second and third days is expected to be similar to that shown in FIG. 4 (near steady state) and similar to that of FIG. 3 thereafter. After about 3 days, fentanyl concentration will come to steady state. The values can be scaled according to the dose strength based on the size differences and that the normalized curves according to dose strength will substantially superimpose.

Example 4: 1-day Patch (with 87-4287 Adhesive), Replaced Daily for 3 or More Days

Monolithic transdermal patches according to FIG. 1 are prepared in 5, 11, 21, 31 and 42 cm$^2$ sizes comprising about 8 wt% each of fentanyl base using the process of Example 1 to correspond to 25 $\mu$g/h, 50 $\mu$g/h, 100 $\mu$g/h, 150 $\mu$g/h, and 200 $\mu$g/h dose strengths.

A polyacrylate adhesive (National Starch DURO-TAK® 87-4287, 100 g) is solubilized in a solvent (ethyl acetate, 160 ml). Fentanyl base is added to the polyacrylate adhesive solution in amounts sufficient to generate a mixture containing 2.8 wt% of fentanyl in the adhesive solution and stirred to dissolve the drug. The solution is cast into reservoir layer and the solvent is evaporated to result in a matrix layer of about 1 mil (0.025mm) thickness. After solvent evaporation, a 1.7 mil thick backing layer comprised of a multilaminate of polyethylene/polyurethane polyester layer is laminated on to the adhesive drug reservoir layer using standard procedures. Individual patches are die-cut from this laminate in 5, 11, 21, 31 and 42 cm$^2$ sizes comprising about 8 wt% each of fentanyl, to generate monolithic transdermal patches containing 0.2 mg/cm$^2$ of fentanyl base as 1-day patches.

Such patches in low doses are to be applied to an opioid-naïve patient with only one patch initially and replaced every 24 hours. Thus, for example, if a patient would receive a 100 $\mu$g/h dose strength device, the administration of the drug to the patient is titrated upward starting with a lower dose strength, e.g., with a 25 $\mu$g/h dose strength. The result is expected to be that the 1-day patches take about 3 days to come to essentially a steady state condition. The $C_{max}$ after 24 hours is only about 50% to 60% that of the steady state $C_{max}$ and the $C_{max}$ after 48 hours is only about 70% to 80% that of the steady state $C_{max}$. The result of the first three days is expected to be similar in shape to that shown in FIG. 5 and similar in shape to that of FIG. 3 at near steady state, after about 3 days, except that the numerical values will be lower than the 100 $\mu$g/h dose strength. The values can be scaled according to the dose strength based on the size differences and that the normalized curves according to dose strength will substantially superimpose.

In another test, such patches are to be applied to an opioid-naive person who has substantially no such drug in the blood, by applying two patches initially at zero hour. Both initial patches are removed at the end of 24 hours and replaced with only one 1-day patch, which is thereafter replaced every 24 hours. The result is expected to be that the 1-day patches takes about 1 day to come to a blood level of essentially steady state condition. The result of the second and third days is expected to be similar in shape to that shown in FIG. 4 (near steady state) and similar in shape to that of FIG. 3 thereafter. The values can be scaled according to the dose strength based on the size differences and that the normalized curves according to dose strength will substantially superimpose.

Example 5: 3 days application:1-day Patch vs. 3-day patches (50 $\mu$g/h)

Monolithic transdermal patches according to FIG. 1 were prepared, which include about 8 wt% of fentanyl base in a process similar to Example 1 using National Starch DURO-TAK® 87-4287 adhesive for 50 $\mu$g/h dose strength. The area of a 3-day patch was 21 cm$^2$ and the fentanyl matrix layer was about 2 mil (0.05mm=50 $\mu$m) thick. Thinner and smaller 16 cm$^2$ patches were also made for the 1-day use patches for comparison. For these thinner patches, the fentanyl solutions were cast to form matrix thicknesses (after solvent evaporation) of 0.038 mm (38 $\mu$m), and 0.025 mm (25 $\mu$m) to form 16 cm$^2$ patches. The 2 mil 3-day monolithic transdermal patches contained 0.4 mg/cm$^2$ of fentanyl base. The 1 mil 1-day monolithic transdermal patches contained 0.2 mg/cm$^2$ of fentanyl base.

Such patches were applied to opioid-naive patients. Each subject was to receive both the 1-day patch and the 3-day patches but not together. Subjects selected were not dependent on opioids. The patches were tested on 18 subjects with different periods for patches of different thicknesses. During each treatment, the study system was worn for the set period on a new application skin site on the application area. For each subject the same anatomical area was used throughout the study. There was a minimum washout period of at least 6 days and not more than 14 days between treatments. The washout period was to commence upon removal of the study system. The 3-day patch (50 $\mu$m) was worn for 72 hours then removed. The 25 $\mu$m patch was worn for 24 hours and changed to a new patch at the 24$^{th}$ and the 48$^{th}$ hour. The 38 $\mu$m patch was worn for 24 hours and changed to a new patch at the 24$^{th}$ and the 48$^{th}$ hour. For one period, a 25 $\mu$m patch was worn for 24 hours, removed and the blood fentanyl concentration was monitored until at the end of the 72nd hour.

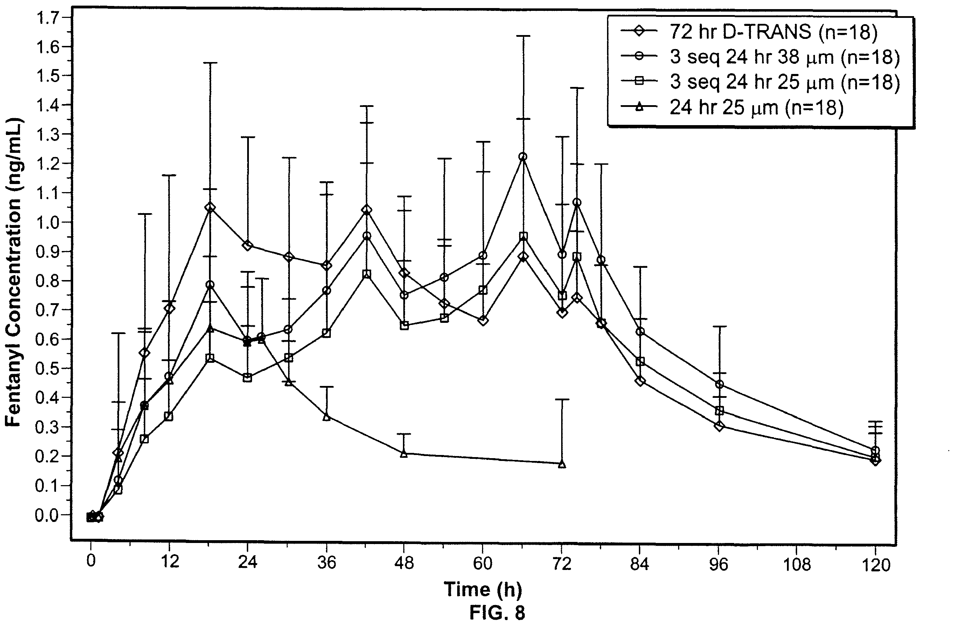

FIG. 8 shows the summary of the averaged data (over all the subjects N=18) on the fentanyl concentration in the blood of the subjects for the various matrix thicknesses. The diamond data points represent the data for the 3-day patches applied for 3 days. The circle data points represent the data for the 1-day 38 $\mu$m patches applied for 3 days with daily replacement. The square data points (below the circle data points) represent the data for the 1-day 25 $\mu$m patches applied for 3 days with daily replacement. The triangle data points represent the data for the 1-day 25 $\mu$m patches where the patches were removed after one day use without replacement. The results show that after three days, the 38 $\mu$m patch might lead to a steady state fentanyl blood content of higher than the 3-day patch could. After three days, the 25 $\mu$m patch led to a steady state fentanyl blood content of about equal to what the 3-day patch steady state blood content would be. The 25 $\mu$m patches at 16 cm$^2$ had only 38% the fentanyl content of the 3-day patch, less than the 52% that was predicted by the Simulation 1 above. These 25 $\mu$m patches had about 0.4 mg/cm$^2$. These patches, with less fentanyl than Simulation 1, were able to meet or exceed the fentanyl delivery rate predicted by Simulation 1.

The mean fentanyl amount delivered was calculated for each treatment by subtracting the mean residual fentanyl content from the initial average fentanyl content of the system at time 0 (t=0) as shown below:

Mean amount delivered (mg)=Initial content (mg) - Mean residual content (mg).

The data show that the mean amount of fentanyl delivered for the 1-day systems was 1.59 mg for the QD 38 $\mu$m system and 1.39 to 1.54 mg for the QD 25 $\mu$m system. The mean amount delivered for the 3-day 50 $\mu$g/h system

was 3.85 mg. For calculation, the initial average fentanyl amounts (actual fentanyl content of the systems at t=0) were 5.47 mg, 3.42 mg, 8.01 mg for the QD 38 $\mu$m, QD 25 $\mu$m, and 3-day (50 $\mu$m) 50 $\mu$g/h systems, respectively. The mean amount delivered during system application represented approximately 29%, 43%, and 48% of the fentanyl in the QD 38 $\mu$m, QD 25 $\mu$m, and 3-day 50 $\mu$g/h systems, respectively. Thus, the thinner 1-day QD patch (25 $\mu$m) was able to have a %-utilization of fentanyl close to the 3-day (50 $\mu$m) system, better than the thicker 1-day (38$\mu$m) QD system.

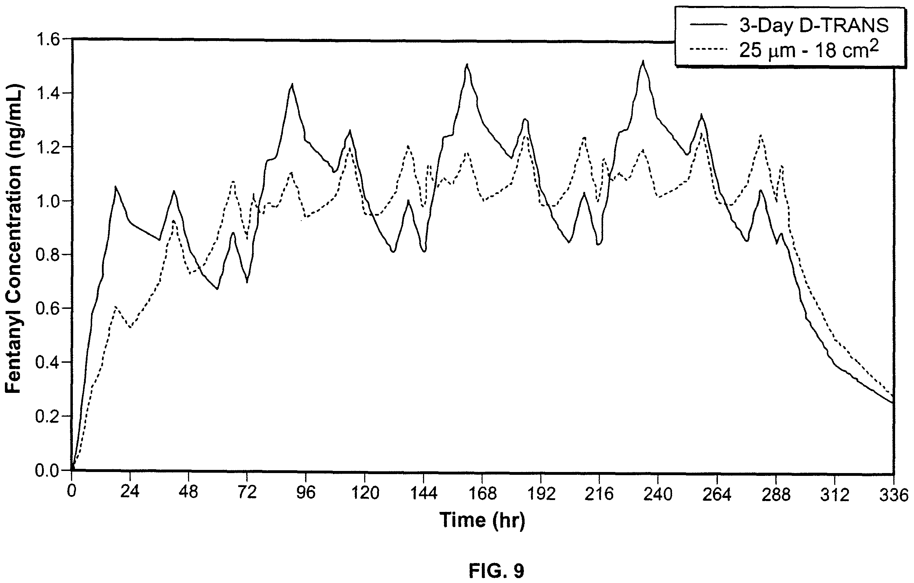

The data for the 50 $\mu$m 3-day patches and the data for the 25 $\mu$m 1-day patches were used to simulate pharmacokinetic profile of steady state profile for using 3-day patches with replacement every 3 days and using 1-day patches with replacement daily using the simulation method of Simulation 1. FIG. 9 shows the simulated data up to 336 hours. The last change of the 3-day patch (solid line) took place at the 216th hour. The last change of the 1-day patch (dashed line) took place at the 264th hour. The simulation data show that the 3-day, 50 $\mu$m, 21 cm² patches had a similar steady state profile as the 1-day, 25 $\mu$m, 18 cm² patches. For dose strengths other than 50 $\mu$g/h, the data information can be scaled for estimation. For example, the 100 $\mu$g/h simulated data can be obtained by doubling the fentanyl concentration of the 50 $\mu$g/h data.

Example 6A: 12 days application:1-day Patch vs. 3-day patches (100 $\mu$g/h)

Monolithic transdermal patches according to FIG. 1 were prepared to contain about 8 wt% of fentanyl base in a process similar to Example 1 using National Starch DURO-TAK® 87-4287 adhesive for 100 $\mu$g/h dose strength. The area of a 3-day patch was 42 cm² and the fentanyl matrix layer was about 2 mil (0.05mm=50 $\mu$m) thick. Thinner 25 $\mu$m thick matrix, 36 cm² patches were also made for the 1-day use patches for comparison. The 1-day patch thus had 43% of the amount of fentanyl of the 3-day patch. These 1-day 25 $\mu$m patches had about 0.2 mg/cm².

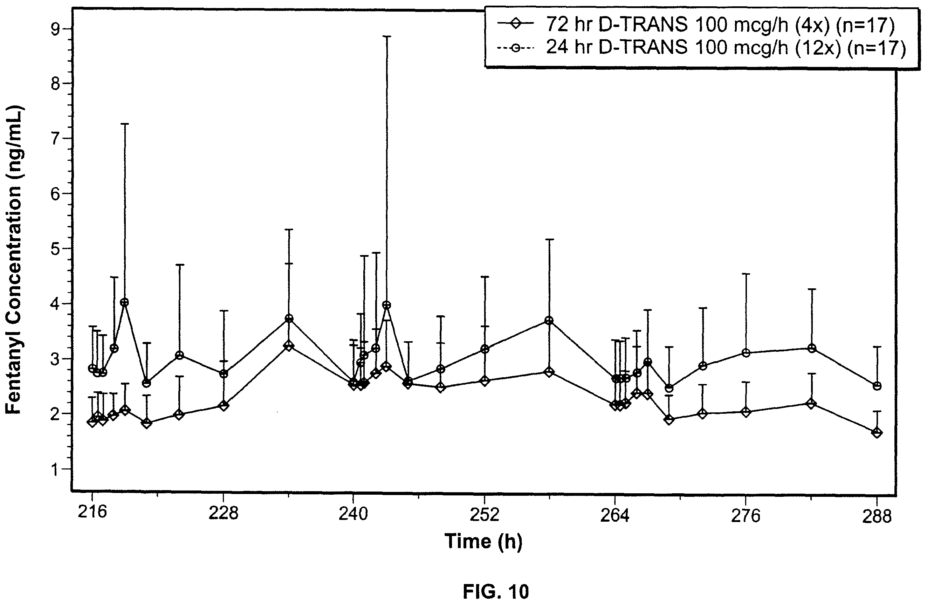

Such patches were applied to opioid-naive patients. Subjects selected were not dependent on opioids. The patches were tested on 17 subjects with different periods for patches of different thicknesses. During each treatment, the study system was worn for the set period on a new application skin site on the application area. For each subject the same area of the body was used throughout the study. There was a minimum washout period of at least 6 days and not more than 14 days between treatments. The washout period was to commence upon removal of the study system. The 3-day 50 $\mu$m patch was worn for 72 hours then removed and replaced with a new patch (total of 4 patches). The 1-day 25 $\mu$m patch was worn for 24 hours then removed and replaced with a new patch (total of 12 patches). Surprisingly, the subjects-averaged (N=17) fentanyl concentration for both sizes continued to increase even after the 144th hour (data before the 216th hour not shown in graphs here). Steady state was reached at or before the 216th hour. FIG. 10 shows the summary of the averaged data (over N=17) on the fentanyl concentration in the blood of the subjects from the 216th hour to the 288th hour. The curve with diamond data points represents the 3-day patches. The curve with open circle data points represents the 1-day patches. The data show that the steady state fentanyl concentration in the blood was higher for the 1-day patches (more than 1.25 as high) compared with the 3-day patches.

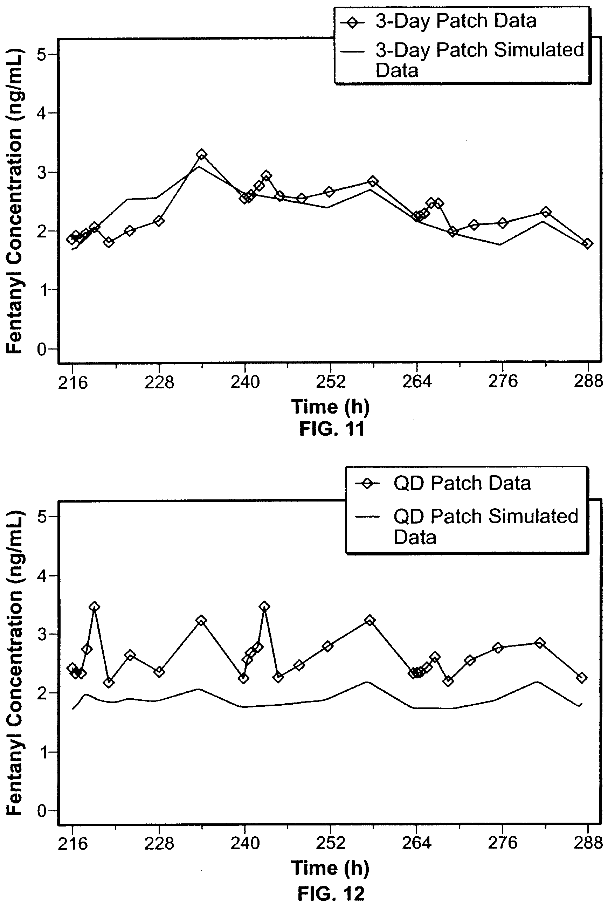

FIG. 11 shows the comparison of the steady state 3-day patch data of FIG. 10 versus the simulated steady state 3-day patch data scaled from the data of FIG. 9 to 100 $\mu$g/h nominal dose strength. The simulated curve is the curve without diamond data points. The two curves match very well. FIG. 12 shows the comparison of the steady state 1-day patch data (the top curve with diamond data points) of FIG. 10 versus the simulated steady state 1-day patch data (bottom curve) scaled from the data of FIG. 9 to 100 $\mu$g/h nominal dose strength. The actual steady state data (the top curve) had an AUC$_{avg}$ of about 29% higher than that of the simulated steady state data. Thus, even though simulation predicted well the results of the 3-day patches, in this case, it did not predict quite as well the results of the 1-day patches.

Example 6B: 12 days application: 1-day Patches

Monolithic transdermal 1-day patches were made according Example 6A. Three dose strengths were used (12.5, 50, and 100 $\mu$g/h).

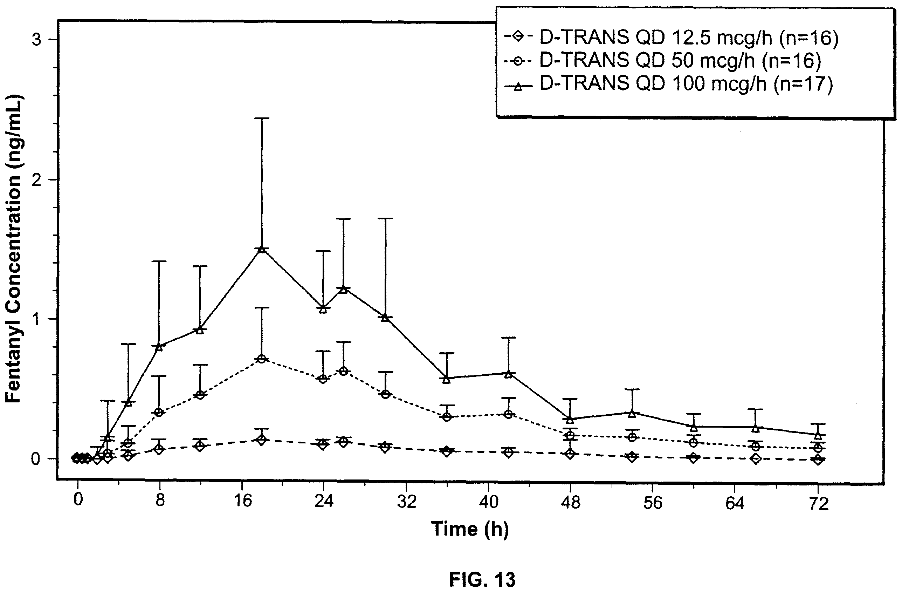

In this study, the dose relationship of a 1-day (called QD for short) fentanyl matrix (25 $\mu$m adhesive thickness) system, with presumed nominal delivery rates of about 12.5 $\mu$g/h, 50 $\mu$g/h, and 100 $\mu$g/h and varying system area size (4.5 cm², 18 cm², and 36 cm², respectively), was evaluated in order to cover the entire dose range of this product. Subjects received the following 3 treatments in a crossover manner according to a randomly assigned sequence: Treatment A: (data points represented in FIG. 13 by diamonds, subjects n=16) fentanyl matrix QD 12.5 $\mu$g/h, 4.5 cm² system area, single application. Treatment B: (data points represented in FIG. 13 by open circles, subjects n=16) fentanyl matrix QD 50 $\mu$g/h, 18 cm² system area, single application. Treatment C: (data points represented in FIG. 13 by open triangles, subjects n=17) fentanyl matrix QD 100 $\mu$g/h, 36 cm² system area, single application. Thirteen subjects completed the study. The 1-day patches thus had about 38% of the amount of fentanyl of the 3-day patches. Such patches were applied to opioid-naive patients. Subjects selected were not dependent on opioids. The patches were tested on the subjects with different periods for patches of different thicknesses. During each treatment, the study system was worn for the set period on a new application skin site on the application area. For each subject the same area of the body was used throughout the study. There was a minimum washout period of at least 6 days and not more than 14 days between treatments. The washout period was to commence upon removal of the study system. Mean serum fentanyl concentrations for each treatment are presented in FIG. 13. The data of FIG. 13 show that the blood fentanyl concentration was a function of the dose strength. The fentanyl blood concentrations, represented by measured fentanyl concentration in serum, when normalized by dividing with the respective dose strength, show curves that were very close to one another in shapes and values.

The initial average fentanyl amounts (measured fentanyl content of the 12.5 $\mu$g/h, 50 $\mu$g/h, and 100 $\mu$g/h systems at t=0) were 0.97 mg, 3.5 mg, and 7.1 mg, respectively. Because all 3 dose strengths (12.5 $\mu$g/h, 50 $\mu$g/h, and 100 $\mu$g/h) were manufactured from the same laminate, they should have the same fentanyl contents on a mg/cm$^2$ basis (0.20 mg/cm$^2$ as the mean of 3 lot release values). The calculated initial fentanyl contents, 0.91 mg, 3.64 mg, and 7.29 mg, for the 12.5 $\mu$g/h, 50 $\mu$g/h, and 100 $\mu$g/h systems, respectively, were then used to calculate the amount of fentanyl delivered. The data showed that the mean amounts of fentanyl delivered for the nominal 12.5 $\mu$g/h, 50 $\mu$g/h, and 100 $\mu$g/h dose strength systems were 0.38 mg, 1.24 mg, and 2.92 mg, respectively. The mean amounts of fentanyl delivered during system application (%-utilization) represented approximately 42%, 34%, and 40% of fentanyl in the 12.5 $\mu$g/h, 50 $\mu$g/h, and 100 $\mu$g/h systems, respectively. In comparison, the %-utilization for DUROGESIC® DTRANS® fentanyl was not much different from these. In a study of DUROGESIC® DTRANS® fentanyl patches, twelve subjects had used systems analyzed for residual fentanyl content after receiving DUROGESIC® DTRANS® fentanyl 100 $\mu$g/h and 12.5 $\mu$g/h (up to 8 systems). The mean residual fentanyl content after receiving the 100 $\mu$g/h fentanyl patches was 9.34 mg. The mean residual fentanyl content after receiving up to 8 patches of 12.5 $\mu$g/h fentanyl was 1.05 mg.

The average fentanyl content for the 100 $\mu$g/h and 12.5 $\mu$g/h DUROGESIC® DTRANS® fentanyl systems at lot clearance was 16.3 and 2.0 mg, respectively. Thus, the average amount of fentanyl absorbed based on the residual fentanyl content is estimated to be 6.96 and 0.95 mg for the 100 $\mu$g/h and 12.5 $\mu$g/h DTRANS® fentanyl systems, respectively. The utilization is 43% and 48% for the 100 $\mu$g/h and 12.5 $\mu$g/h dose strength patch, respectively. Of course, the %-utilization values for the DUROGESIC® DTRANS® fentanyl systems were values after 3 days of use. The average %-utilization per day of use for the DUROGESIC® DTRANS® fentanyl systems was only 1/3 of the 3-day values and would be about 12.5% and 16% for the 100 $\mu$g/h and 12.5 $\mu$g/h dose strength patch, respectively.

Example 7: 12 days application:1-day 32 cm$^2$ Patch vs. 3-day patches (100 $\mu$g/h)

Monolithic transdermal patches according to FIG. 1 were prepared comprising about 8 wt% of fentanyl base in a process similar to Example 1 using National Starch DURO-TAK® 87-4287 adhesive for 100 $\mu$g/h dose strength. The area of a 3-day patch was 42 cm$^2$ and the fentanyl matrix layer was about 2 mil (0.05mm=50 $\mu$m) thick. Thinner 25 $\mu$m, 32 cm$^2$ patches were also made for the 1-day use patches for comparison. The 1-day patches thus had about 38% of the amount of fentanyl of the 3-day patches.

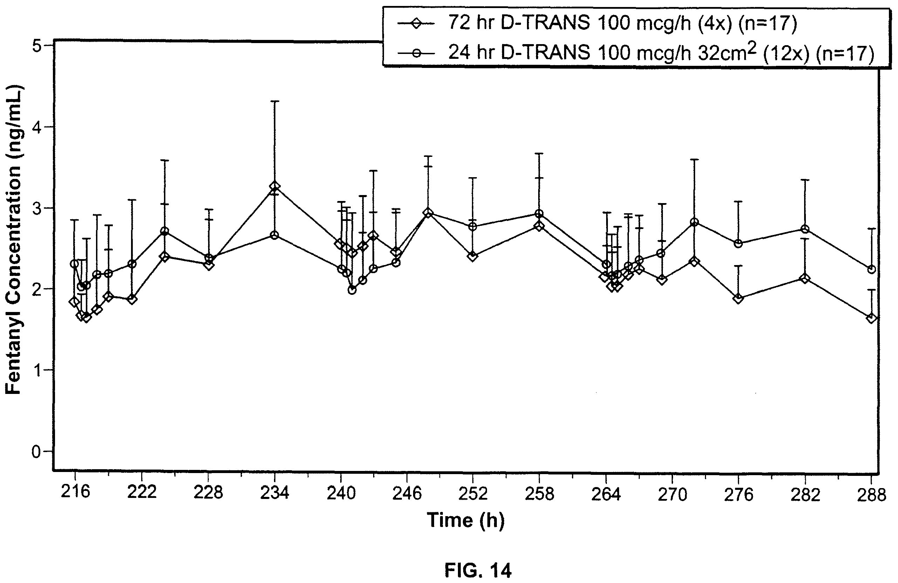

Such patches were applied to opioid-naïve patients. Subjects selected were not dependent on opioids. The patches were tested on 17 subjects with different periods for patches of different thicknesses. During each treatment, the study system was worn for the set period on a new application skin site on the application area. For each subject the same area of the body was used throughout the study. There was a minimum washout period of at least 6 days and not more than 14 days between treatments. The washout period was to commence upon removal of the study system. The 3-day 50 $\mu$m patch was worn for 72 hours then removed and replaced with a new patch (total of 4 patches). The 1-day 25 $\mu$m patch was worn for 24 hours then removed and replaced with a new patch (total of 12 patches). Data were collected to the 360th hours. The subjects-averaged (N=17) fentanyl concentration for both sizes continued to increase even after the 144th hour. Steady state was reached at or before the 216th hour. FIG. 14 shows the summary of the averaged data (over N=17) on the fentanyl concentration in the blood of the subjects from the 216th hour to the 288th hour. The curve with the open circle data points represents the 1-day patches. The curve with the diamond data points represents the 3-day patches. The data show that the steady state AUC fentanyl concentration in the blood was slightly higher for the 1-day patches compared with the 3-day patches.

Table 2 shows the comparison of the pharmacokinetic parameters of the 3-day patches versus those of the 1-day patches. For the steady state condition, data were considered from the 216th hour to the 288th hour for a period of 72 hours. AUC$_{ss}$ data are presented as geometric mean and mean (%CV). CV is standard deviation divided by the mean. C$_{max}$ and C$_{min}$ are presented as mean (%CV).

The AUC$_{ss}$ of the 1-day patch (Trt C) was about 1.07 that of the 3-day patch (Trt A). The C$_{max}$ of the 1-day patch was about 0.94 that of the 3-day patch. The C$_{min}$ of the 1-day patch was about 1.2 that of the 3-day patch. Thus, the 1-day patch was bioequivalent to the 3-day patch. In Table 2, a striking difference is the fluctuation, defined as (C$_{max}$ - C$_{min}$)/C$_{avg}$, where in this case C$_{avg}$= AUC$_{ss}$/72. The fluctuation is the indication of how much the blood fentanyl concentration fluctuates during steady state, expressed in a dimensionless number. In the case of the 3-day patch, the swing between C$_{max}$ and C$_{min}$ was almost as large as the average concentration C$_{avg}$. In the case of the 1-day patch, the fluctuation was only about 64%, substantially smaller than that of the 3-day patch. Thus, the 1-day patch provided a more steady blood concentration.

Table 2 Mean (CV%) Pharmacokinetic Parameters

| Treatment | AUCss (ng.h/mL) | Cmax,ss (ng/mL) | Cmin,ss (ng/mL) | Fluctuation (%) |
|---|---|---|---|---|
| Trt A 3-Day | 171 (15) | 3.6 (23) | 1.5 (15) | 90.2 (28) |
| Trt C 1-Day | 184 (20) | 3.4 (22) | 1.8 (16) | 63.8 (22) |

Table 3 shows the statistics of the data of the 3-day patch (Trt A) versus those of the 1-day patch (Trt C).

TABLE 3

| Parameter | Contrast | Ratio (%) | P Value | 90% Conf. Interval | |
|---|---|---|---|---|---|
| | | | | Lower | Upper |
| AUC(216-288) | Trt C / Trt A | 106 | 0.215 | 97.81 | 115.94 |
| Cmin | Trt C / Trt A | 120 | <0.001 | 112.85 | 128.77 |
| Cmax | Trt C / Trt A | 94 | 0.319 | 85.38 | 103.58 |

Thus, the statistics demonstrate that the 90% confidential interval of $C_{max}$, and $AUC_{ss}$ ratios of the 1-day patch to DUROGESIC® DTRANS® were within 80% and 125%. Therefore, the 1-day patch is bioequivalent to the 3-day patch, DUROGESIC® DTRANS®, 100 $\mu$g/h. The 32 cm$^2$ 1-day patch contains 6.7 mg fentanyl and the DUROGESIC® DTRANS® 100 $\mu$g/h patch contains 16.8 mg fentanyl. Therefore, it takes three 1-day patches (a total of 20.1 mg fentanyl to deliver an bioequivalent amount of fentanyl from a 3-day patch containing 16.8 mg fentanyl, about a 18.5% difference. From a %-utilization perspective, it is a drop from 43% to 35%. However, this performance of %-utilization of the 1-day patch is reasonably good considering it had only 1/3 the time of the 3-day patch to use the fentanyl in the patch compared to 3-day patches. From a %-utilization per day perspective, the 1-day patch had a much higher %-utilization than the 3-day patch. Considering individual patches, the residual fentanyl per patch in the 1-day patch is significantly lower than the 3-day patches.

Example 8: 12 days application: 1-day 28 cm$^2$ Patch vs. 3-day patches (100 $\mu$g/h)

Monolithic transdermal patches according to FIG. 1 were including comprising about 8 wt% of fentanyl base in a process similar to Example 1 using National Starch DURO-TAK® 87-4287 adhesive for 100 $\mu$g/h dose strength. The area of a 3-day patch was 42 cm$^2$ and the fentanyl matrix layer was about 2 mil (0.05mm=50 $\mu$m) thick. Thinner 25 $\mu$m, 28 cm$^2$ patches were also made for the 1-day use patches for comparison. The 1-day patches thus had about 33% of the amount of fentanyl of the 3-day patches.

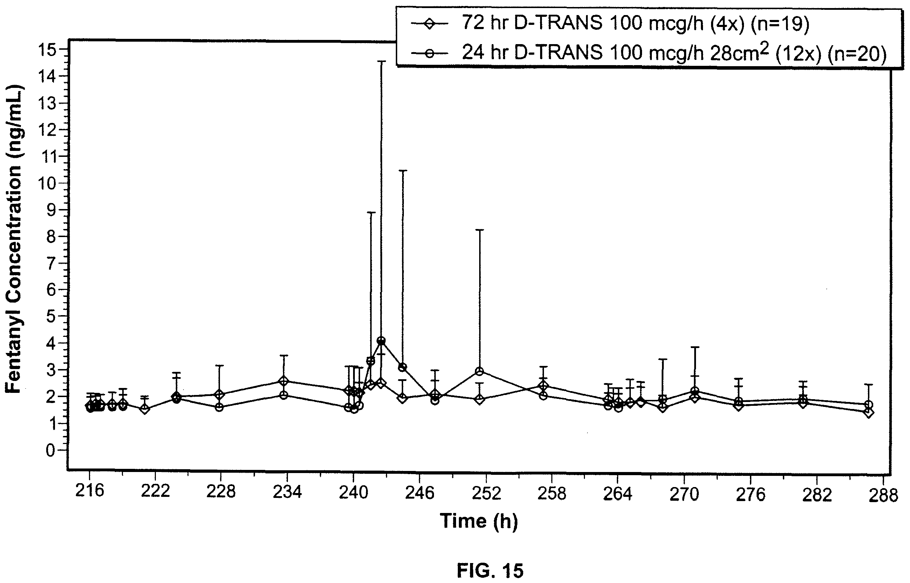

Such patches were applied to opioid-naive patients. Subjects selected were not dependent on opioids. The patches were tested on 19 to 20 subjects with different periods for patches of different thicknesses. During each treatment, the study system was worn for the set period on a new application skin site on the application area. For each subject the same area of the body was used throughout the study. There was a minimum washout period of at least 6 days and not more than 14 days between treatments. The washout period was to commence upon removal of the study system. The 3-day 50 $\mu$m patch was worn for 72 hours then removed and replaced with a new patch (total of 4 patches). The 1-day 25 $\mu$m patch was worn for 24 hours then removed and replaced with a new patch (total of 12 patches). Data were collected to the 260th hour. The subjects-averaged fentanyl concentration for both sizes continued to increase even after the 144th hour. Steady state was reached at or before the 216th hour. FIG. 15 shows the summary of the averaged data on the fentanyl concentration in the blood of the subjects from the 216th hour to the 288th hour. The data show that the steady state $AUC_{ss}$ fentanyl concentration in the blood was close to that for the 1-day patches (n=20) compared with the 3-day patches (n=19). One of the 20 subjects was not tested with 3-day patches. The diamonds represent the data points for the 3-day patches. The open circles represent the data points for the 1-day patches. In FIG. 15, although the $AUC_{ss}$ were close for the two types of patches, there were four data points (e.g., at 242, 243, 245, 252 hour) that had large differences among the individual subjects, thus affecting the statistical significance of the data.

Table 4A and Table 4B show the comparison of the pharmacokinetic parameters of the 3-day patches versus those of the 1-day patches. For the steady state condition, data were considered from the 216th hour to the 288th hour for a period of 72 hours. $AUC_{ss}$ data are presented as geometric mean and mean (%CV). CV is standard deviation divided by the mean. $C_{max}$ and $C_{min}$ are presented as mean (%CV).

Table 4A has data for 19 subjects (N=19) on which both 1-day patches and 3-day patches were separately applied. Thus, the subject on whom only 1-day patches were applied (but without separately applying 3-day patches) was excluded.

TABLE 4A Mean (CV%) Pharmacokinetic Parameters

| Treatment | AUCss (ng.h/mL) | Cmax,ss (ng/mL) | Cmin,ss (ng/mL) | Fluctuation (%) |
|---|---|---|---|---|
| Trt A 3-Day | 147 (30) | 3.0 (35) | 1.3 (24) | 79.3 (30) |
| Trt C 1-Day | 149 (62) | 6.1 (186) | 1.3(31) | 158 (164) |

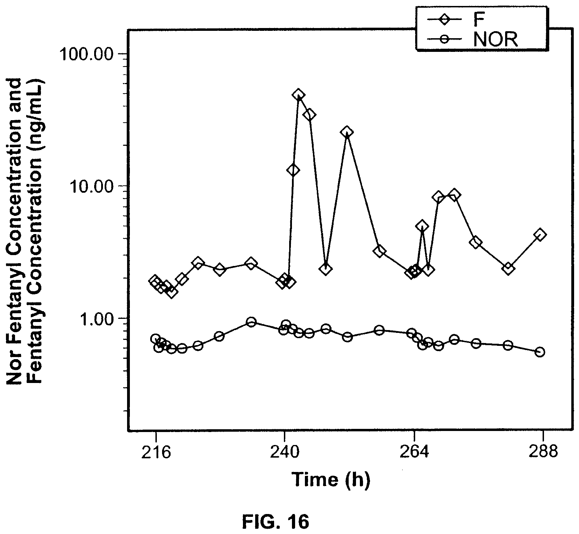

Five of the 19 subjects had outlier data points (i.e., data points that differed significantly from neighboring data points). The analysis for fentanyl metabolite norfentanyl in their samples collected between 216th and 288th hr showed that the metabolite concentrations were stable for all 5 subjects that had outlier data, indicating the outlier data for 5 subjects were not due to transdermal fentanyl transport. The large fluctuation was likely due to contamination in certain samples of 5 subjects that were analyzed. For example, FIG. 16 shows the serum concentration of fentanyl and norfentanyl of an exemplary subject who had outlier data. Curve F with the diamond data points represents the 1-day patch fentanyl data, whereas curve NOR with the open circle data points represents the norfentanyl data of the same patch delivery. Some of the data points after 240 hours had wide variations in fentanyl concentration whereas the metabolite norfentanyl concentration was stable for the same time period. It is physically impossible for the serum fentanyl concentration to fluctuate so widely and so rapidly by transdermal delivery. Thus, at that period, the norfentanyl concentration was a better indicator of the variation of the amount of fentanyl delivered. Table 4B shows the data of Table 4A after the exclusion of data from subjects having outlier numbers. The number of subjects in Table 4B is 14 (N=14).

TABLE 4B Mean (CV%) Pharmacokinetic Parameters

| Treatment | AUCss (ng.h/mL) | Cmax,ss (ng/mL) | Cmin,ss (ng/mL) | Fluctuation (%) |
|---|---|---|---|---|
| Trt A 3-Day | 147 (33) | 3.0 (39) | 1.3 (25) | 75 (33) |
| Trt C 1-Day | 132 (30) | 2.6 (33) | 1.3 (32) | 70 (38) |

Table 4B shows that the $AUC_{ss}$ (shown by $AUC_{(216-288)}$ from the 216th hour to the 218th hour) of the 1-day patch was about 0.9 that of the 3-day patch based on the geometric mean data. The geometric mean of the $C_{max}$ of the 1-day patch was about 0.9 that of the 3-day patch. The geometric mean $C_{min}$ of the 1-day patch was about 1.0 that of the 3-day patch. The amount of fluctuation (in %) was about 70 for the 1-day patch versus about 75 for the 3-day patch. If only Table 4B is considered, because the $AUC_{ss}$, $C_{max}$ and $C_{min}$ were close between the 1-day patch and the 3-day patch in Table 4B, the 1-day patches can be considered to be bioequivalent to the 3-day patch. From the Table 4B data, the 1-day 28 cm$^2$ patch provided a bioequivalent amount of fentanyl to the body as DUROGESIC® DTRANS® 100 $\mu$g/h. The amount of fentanyl in each 28 cm$^2$ patch is about 5.9 mg. Therefore, it takes total 17.7 mg (3x5.9) fentanyl in 1-day patch to deliver the same amount of fentanyl as 16.8 mg of fentanyl in a DUROGESIC® DTRANS® 100 $\mu$g/h patch. The utilization for a 1-day 28 cm$^2$ patch after use was estimated to be 39%, which was only slightly below that of the 43% for the DUROGESIC® DTRANS® 100 $\mu$g/h patch. However, the utilization per day was significantly higher in the 1-day patch.

The practice of the present invention will employ, unless otherwise indicated, conventional methods used by those in pharmaceutical product development within those of skill of the art. Such techniques are explained fully in the literature.

**Claims**

1. A kit for administering a drug with a transdermal patch, comprising:

   (a) a transdermal patch for administration of fentanyl base through the skin, comprising:

   a backing layer;
   a reservoir disposed on the backing layer, at least the skin contacting surface of the reservoir being adhesive;
   the reservoir comprising a polymeric composition containing polyacrylate and an amount of fentanyl base sufficient to induce and maintain analgesia in a human for one day; and

(b) an instruction print including instruction on daily replacement of the transdermal patch,
wherein the normalized area based on nominal dose strength is 0.2 to 0.4cm$^2$ per $\mu$g/h per day and the fentanyl base loading per surface is 0.14 to 0.3 mg/cm$^2$.

2. The kit of claim 1 wherein the reservoir comprises an amount of dissolved fentanyl base at a concentration of from 0.14 to 0.3 mg/cm$^2$, optionally the reservoir:

i) comprises a polymer having a solubility for fentanyl base of 1 wt% to 25 wt%; or
ii) comprises from 0.14 to 0.3 mg/cm$^2$ of fentanyl base per area of the reservoir and the patch has an area normalized to dose strength of 10.5 to 15 cm$^2$ per mg fentanyl nominally for 1-day delivery; or
iii) has a thickness of 0.0125 mm (0.5 mil) to 0.0375 mm (1.5mil), and optionally further comprises an enhancer.

3. The kit of claim 1 wherein the patch has an area normalized to dose strength of 8.5 to 16.5 cm$^2$ per mg fentanyl nominally for 1-day delivery.

4. The kit of claim 1 wherein there is only one adhesive or matrix layer in the transdermal patch and the reservoir contains no enhancer.

5. The kit of claim 1 wherein the backing layer comprises a polymer selected from at least one of the following: polyurethane, polyvinyl acetate, polyvinylidene chloride, polyethylene, polyethylene terephthalate (PET), polyester, ethylene vinyl acetate, and polybutylene terephthalate.

6. The kit of claim 1 wherein the backing layer has a thickness of 0.012 mm (0.5 mil) to 0.125 mm (5 mil).

7. The kit of claim 1 wherein the reservoir is a matrix reservoir having a polyacrylate matrix, the polyacrylate having 5.2 wt% 2-hydroxyethyl acrylate monomer, 20-40 wt% vinyl acetate, and 55-75 wt% 2-ethylhexyl acrylate.

8. A transdermal patch for administering of fentanyl base through the skin, comprising:

a backing layer;
a reservoir disposed on the backing layer, at least the skin contacting surface of the reservoir being adhesive;
the reservoir comprising a polymeric composition containing polyacrylate and an amount of fetanyl base sufficient to induce and maintain analgesia in a human for one day,
wherein the normalized area based on nominal dose strength is 0.2 to 0.4cm$^2$ per $\mu$g/h per day and the fentanyl base loading per surface area is 0.14 to 0.3 mg/cm$^2$

9. Fentanyl base for use in a method of treating pain, the method comprising replacing one monolithic transdermal patch daily on the skin of a human in need thereof, wherein the patch contains a backing and a reservoir comprising a polymeric composition containing polyacrylate and fentanyl base, wherein the normalized area based on nominal dose strength is 0.2 to 0.4cm$^2$ per $\mu$g/h per day and the fentanyl base loading per surface area is 0.14 to 0.3 mg/cm$^2$.

10. Fentanyl base for use in a method as claimed in claim 9, wherein said method further comprises determining that the human is opioid naive and applying only one patch at first and daily replacement of one patch.

11. Fentanyl base for use in a method as claimed in claim 9, wherein said method further comprises determining that the human is opioid naive and applying one patch at first and daily applying a new patch to replace a day-old patch such that a fentanyl base level in plasma is at a steady state range in two days.

12. Fentanyl base for use in a method as claimed in claim 9, wherein said method further comprises determining that the human is opioid tolerant on day one of patch application and applying two patches at first and daily applying a new patch to replace a day-old patch starting from day two, such that a fentanyl base level in plasma is effective for analgesia in one day.

13. Fentanyl base for use in a method as claimed in claim 9, wherein said method further comprises determining that according to the human's medical record that the human is opioid tolerant at first of patch application and applying two patches at first and daily applying a new patch to replace a day-old patch starting from day two, such that a fentanyl base level in plasma is effective for analgesia within one day.

14. Fentanyl base for use in a method as claimed in claim 9, wherein the reservoir in said method comprises an amount of dissolved fentanyl base at a concentration of from 20 mg/gm to 200 mg/gm.

15. Fentanyl base for use in a method as claimed in claim 9, wherein the reservoir in said method comprises a polymer having a solubility for fentanyl base of 1 wt% to 25 wt%.

16. Fentanyl base for use in a method as claimed in claim 9, wherein the reservoir in said method comprises 0.14 to 0.3 mg/cm$^2$ of fentanyl base per area of the reservoir and the patch has an area normalized to dose strength of 10.5 to 15 cm$^2$ per mg fentanyl base nominally for 1-day delivery.

17. Fentanyl base for use in a method as claimed in claim 9 wherein the reservoir in said method has a thickness of from 0.0125 mm (0.5 mil) to 0.0375 mm (1.5 mil).

18. The kit according to claim 1, the transdermal patch according to claim 8 or the fentanyl base for use in a method as claimed in claim 9, wherein the fentanyl base content per patch is 4 to 8 mg.


**Patentansprüche**

1. Kit zur Verabreichung eines Arzneimittels mit einem transdermalen Pflaster, umfassend:

    (a) ein transdermales Pflaster zur Verabreichung von Fentanyl-Base durch die Haut, umfassend:

    eine Trägerschicht;
    ein auf der Trägerschicht angeordnetes Reservoir, wobei wenigstens die die Haut berührende Oberfläche des Reservoirs klebend ist; wobei das Reservoir eine polymere Zusammensetzung umfasst, die Polyacrylat und eine Menge Fentanyl-Base enthält, die ausreicht, um bei einem Menschen Analgesie für einen Tag zu induzieren und zu erhalten; und

    (b) eine gedruckte Anleitung, die eine Anleitung zum täglichen Austausch des transdermalen Pflasters enthält, wobei die auf der nominalen Dosisstärke basierende normalisierte Fläche 0,2 bis 0,4 cm$^2$ pro μg/h pro Tag beträgt und die Fentanyl-Base-Beladung pro Oberfläche 0,14 bis 0,3 mg/cm$^2$ beträgt.

2. Kit nach Anspruch 1, wobei das Reservoir eine Menge gelöster Fentanyl-Base in einer Konzentration von 0,14 bis 0,3 mg/cm$^2$ umfasst, wobei das Reservoir wahlweise:

    (i) ein Polymer mit einer Löslichkeit für Fentanyl-Base von 1 Gew.-% bis 25 Gew.-% umfasst; oder
    (ii) 0,14 bis 0,3 mg/cm$^2$ Fentanyl-Base pro Fläche des Reservoirs umfasst und das Pflaster eine zur Dosisstärke normalisierte Fläche von 10,5 bis 15 cm$^2$ pro mg Fentanyl nominal zur 1-tägigen Abgabe umfasst; oder
    (iii) eine Dicke von 0,0125 mm bis 0,0375 mm aufweist, und wahlweise ferner einen Verstärker umfasst.

3. Kit nach Anspruch 1, wobei das Pflaster eine zur Dosisstärke normalisierte Fläche von 8,5 bis 16,5 cm$^2$ pro mg Fentanyl nominal zur 1-tägigen Abgabe aufweist.

4. Kit nach Anspruch 1, wobei es in dem transdermalen Pflaster nur eine Klebe- oder Matrixschicht gibt und das Reservoir keinen Verstärker enthält.

5. Kit nach Anspruch 1, wobei die Trägerschicht ein Polymer umfasst, das aus wenigstens einem der folgenden ausgewählt ist: Polyurethan, Polyvinylacetat, Polyvinylidenchlorid, Polyethylen, Polyethylenterephthalat (PET), Polyester, Ethylenvinylacetat, und Polybutylenterephthalat.

6. Kit nach Anspruch 1, wobei die Trägerschicht eine Dicke von 0,012 mm bis 0,125 mm aufweist.

7. Kit nach Anspruch 1, wobei das Reservoir ein Matrixreservoir mit einer Polyacrylatmatrix ist, wobei das Polyacrylat 5,2 Gew.-% 2-Hydroxyethylacrylatmonomer, 20-40 Gew.-% Vinylacetat, und 55-75 Gew.-% 2-Ethylhexylacrylat aufweist.

8. Transdermales Pflaster zur Verabreichung von Fentanyl-Base durch die Haut, umfassend:

eine Trägerschicht;

ein auf der Trägerschicht angeordnetes Reservoir, wobei wenigstens die die Haut berührende Oberfläche des Reservoirs klebend ist; wobei das Reservoir eine polymere Zusammensetzung umfasst, die Polyacrylat und eine Menge Fentanyl-Base enthält, die ausreicht, um bei einem Menschen Analgesie für einen Tag zu induzieren und zu erhalten; und

wobei die auf der nominalen Dosisstärke basierende normalisierte Fläche 0,2 bis 0,4 cm$^2$ pro $\mu$g/h pro Tag beträgt und die Fentanyl-Base-Beladung pro Oberfläche 0,14 bis 0,3 mg/cm$^2$ beträgt.

**9.** Fentanyl-Base zur Verwendung in einem Verfahren zur Schmerzbehandlung, wobei das Verfahren den Austausch eines monolithischen transdermalen Pflasters pro Tag auf der Haut eines Menschen, der dessen bedarf, umfasst, wobei das Pflaster eine Trägerschicht enthält sowie ein Reservoir, das eine polymere Zusammensetzung umfasst, die Polyacrylat und Fentanyl-Base enthält, wobei die auf der nominalen Dosisstärke basierende normalisierte Fläche 0,2 bis 0,4 cm$^2$ pro $\mu$g/h pro Tag beträgt und die Fentanyl-Base-Beladung pro Oberfläche 0,14 bis 0,3 mg/cm$^2$ beträgt.

**10.** Fentanyl-Base zur Verwendung in einem Verfahren nach Anspruch 9, wobei das Verfahren ferner umfasst, dass bestimmt wird, dass der Mensch opioidnaiv ist, sowie das Anwenden von zuerst nur einem Pflaster und den täglichen Austausch eines Pflasters.

**11.** Fentanyl-Base zur Verwendung in einem Verfahren nach Anspruch 9, wobei das Verfahren ferner umfasst, dass bestimmt wird, dass der Mensch opioidnaiv ist, sowie das Anwenden von zuerst einem Pflaster und tägliches Anwenden eines neuen Pflasters, um ein einen Tag altes Pflaster zu ersetzen, sodass ein Fentanyl-Base-Spiegel im Plasma sich in zwei Tagen in einem Gleichgewichtsbereich befindet.

**12.** Fentanyl-Base zur Verwendung in einem Verfahren nach Anspruch 9, wobei das Verfahren ferner umfasst, dass am ersten Tag der Pflasteranwendung bestimmt wird, dass der Mensch opioidtolerant ist, sowie das Anwenden von zuerst zwei Pflastern und beginnend mit dem zweiten Tag tägliches Anwenden eines neuen Pflasters, um ein einen Tag altes Pflaster zu ersetzen, sodass ein Fentanyl-Base-Spiegel im Plasma zur Analgesie in einem Tag wirksam ist.

**13.** Fentanyl-Base zur Verwendung in einem Verfahren nach Anspruch 9, wobei das Verfahren ferner umfasst, dass bei der ersten Pflasteranwendung bestimmt wird, dass der Mensch nach seiner Patientenakte opioidtolerant ist, sowie das Anwenden von zuerst zwei Pflastern und beginnend mit dem zweiten Tag tägliches Anwenden eines neuen Pflasters, um ein einen Tag altes Pflaster zu ersetzen, sodass ein Fentanyl-Base-Spiegel im Plasma zur Analgesie innerhalb eines Tages wirksam ist.

**14.** Fentanyl-Base zur Verwendung in einem Verfahren nach Anspruch 9, wobei das Reservoir in dem Verfahren eine Menge gelöster Fentanyl-Base in einer Konzentration von 20 mg/g bis 200 mg/g umfasst.

**15.** Fentanyl-Base zur Verwendung in einem Verfahren nach Anspruch 9, wobei das Reservoir in dem Verfahren ein Polymer mit einer Löslichkeit für Fentanyl-Base von 1 Gew.-% bis 25 Gew.-% umfasst.

**16.** Fentanyl-Base zur Verwendung in einem Verfahren nach Anspruch 9, wobei das Reservoir in dem Verfahren 0,14 bis 0,3 mg/cm$^2$ Fentanyl-Base pro Fläche des Reservoirs umfasst und das Pflaster eine zur Dosisstärke normalisierte Fläche von 10,5 bis 15 cm$^2$ pro mg Fentanyl-Base nominal zur 1-tägigen Abgabe aufweist.

**17.** Fentanyl-Base zur Verwendung in einem Verfahren nach Anspruch 9, wobei das Reservoir in dem Verfahren eine Dicke von 0,0125 mm bis 0,0375 mm aufweist.

**18.** Kit nach Anspruch 1, transdermales Pflaster nach Anspruch 8 oder die Fentanyl-Base zur Verwendung in einem Verfahren nach Anspruch 9, wobei der Fentanyl-Base-Gehalt pro Pflaster 4 bis 8 mg beträgt.

**Revendications**

**1.** Trousse pour administrer un médicament avec un timbre transdermique, comprenant :

(a) un timbre transdermique pour administration de fentanyl base à travers la peau, comprenant :

une couche de support ;

un réservoir disposé sur la couche de support, au moins la surface en contact avec la peau du réservoir étant adhésive ; le réservoir comprenant une composition polymère contenant du polyacrylate et une quantité de fentanyl base suffisante pour induire et maintenir une analgésie chez un humain pendant un jour ; et

(b) une impression d'instruction comprenant une instruction sur le remplacement quotidien du timbre transdermique,

dans laquelle l'aire normalisée sur la base de la dose nominale est de 0,2 à 0,4 cm$^2$ par µg/h par jour et la charge de fentanyl base par surface est de 0,14 à 0,3 mg/cm$^2$.

2. Trousse selon la revendication 1, dans laquelle le réservoir comprend une quantité de fentanyl base dissous à une concentration de 0,14 à 0,3 mg/cm$^2$, facultativement, le réservoir :

i) comprend un polymère ayant une solubilité pour le fentanyl base de 1 % en poids à 25 % en poids ; ou
ii) comprend de 0,14 à 0,3 mg/cm$^2$ de fentanyl base par aire du réservoir et le timbre a une aire normalisée par rapport à la dose de 10,5 à 15 cm$^2$ par mg de fentanyl nominale pour administration pendant 1 jour ; ou
iii) a une épaisseur de 0,0125 mm (0,5 mil) à 0,0375 mm (1,5 mil), et comprend facultativement en outre un adjuvant.

3. Trousse selon la revendication 1, dans laquelle le timbre a une aire normalisée par rapport à la dose de 8,5 à 16,5 cm$^2$ par mg de fentanyl nominale pour administration pendant 1 jour.

4. Trousse selon la revendication 1, dans laquelle il y a une seule couche d'adhésif ou de matrice dans le timbre transdermique et le réservoir ne contient aucun adjuvant.

5. Trousse selon la revendication 1, dans laquelle la couche de support comprend un polymère choisi parmi au moins l'un des suivants : polyuréthane, poly(acétate de vinyle), poly(chlorure de vinylidène), polyéthylène, poly(téréphtalate d'éthylène) (PET), polyester, éthylène-acétate de vinyle et poly(téréphtalate de butylène).

6. Trousse selon la revendication 1, dans laquelle la couche de support a une épaisseur de 0,012 mm (0,5 mil) à 0,125 mm (5 mil).

7. Trousse selon la revendication 1, dans laquelle le réservoir est un réservoir de matrice ayant une matrice de polyacrylate, le polyacrylate ayant 5,2 % en poids de monomère d'acrylate de 2-hydroxyéthyle, 20 à 40 % en poids d'acétate de vinyle, et 55 à 75 % en poids d'acrylate de 2-éthylhexyle.

8. Timbre transdermique pour l'administration de fentanyl base à travers la peau, comprenant :

une couche de support ;
un réservoir disposé sur la couche de support, au moins la surface en contact avec la peau du réservoir étant adhésive ; le réservoir comprenant une composition de polymère contenant du polyacrylate et une quantité de fentanyl base suffisante pour induire et maintenir une analgésie chez un humain pendant un jour,
dans laquelle l'aire normalisée sur la base de la dose nominale est de 0,2 à 0,4 cm$^2$ par µg/h par jour et la charge de fentanyl base par surface est de 0,14 à 0,3 mg/cm$^2$.

9. Fentanyl base pour utilisation dans un procédé de traitement de la douleur, le procédé comprenant le remplacement d'un timbre transdermique monolithique chaque jour sur la peau d'un humain en ayant besoin, le timbre contenant un support et un réservoir comprenant une composition de polymère contenant du polyacrylate et du fentanyl base, dans laquelle l'aire normalisée sur la base de la dose nominale est de 0,2 à 0,4 cm$^2$ par µg/h par jour et la charge de fentanyl base par surface est de 0,14 à 0,3 mg/cm$^2$.

10. Fentanyl base pour utilisation dans un procédé selon la revendication 9, ledit procédé comprenant en outre la détermination que l'humain est naïf aux opioïdes et l'application d'un seul timbre initialement et le remplacement quotidien par un timbre.

11. Fentanyl base pour utilisation dans un procédé selon la revendication 9, ledit procédé comprenant en outre la détermination que l'humain est naïf aux opioïdes et l'application d'un timbre initialement et l'application quotidienne d'un timbre neuf pour remplacer un timbre âgé d'un jour de sorte qu'un taux de fentanyl base dans le plasma est

EP 2 207 507 B1

dans une plage à l'équilibre en deux jours.

**12.** Fentanyl base pour utilisation dans un procédé selon la revendication 9, ledit procédé comprenant en outre la détermination que l'humain est tolérant aux opioïdes au jour un de l'application de timbre et l'application de deux timbres initialement et l'application quotidienne d'un timbre neuf pour remplacer un timbre âgé d'un jour à partir du jour deux, de sorte qu'un taux de fentanyl base dans le plasma soit efficace pour l'analgésie en un jour.

**13.** Fentanyl base pour utilisation dans un procédé selon la revendication 9, ledit procédé comprenant en outre la détermination que, selon le dossier médical de l'humain, l'humain est tolérant aux opioïdes au début de l'application de timbre et l'application de deux timbres initialement et l'application quotidienne d'un timbre neuf pour remplacer un timbre âgé d'un jour à partir du jour deux, de sorte qu'un taux de fentanyl base dans le plasma soit efficace pour l'analgésie en un jour.

**14.** Fentanyl base pour utilisation dans un procédé selon la revendication 9, le réservoir dans ledit procédé comprenant une quantité de fentanyl base dissous à une concentration de 20 mg/g à 200 mg/g.

**15.** Fentanyl base pour utilisation dans un procédé selon la revendication 9, le réservoir dans ledit procédé comprenant un polymère ayant une solubilité pour le fentanyl base de 1 % en poids à 25 % en poids.

**16.** Fentanyl base pour utilisation dans un procédé selon la revendication 9, le réservoir dans ledit procédé comprenant 0,14 à 0,3 mg/cm$^2$ de fentanyl base par aire du réservoir et
le timbre a une aire normalisée par rapport à la dose de 10,5 à 15 cm$^2$ par mg de fentanyl base nominale pour administration pendant 1 jour.

**17.** Fentanyl base pour utilisation dans un procédé selon la revendication 9, le réservoir dans ledit procédé ayant une épaisseur de 0,0125 mm (0,5 mil) à 0,0375 mm (1,5 mil) .

**18.** Trousse selon la revendication 1, timbre transdermique selon la revendication 8 ou fentanyl base pour utilisation dans un procédé selon la revendication 9, la teneur en fentanyl base par timbre est de 4 à 8 mg.

**FIG. 1**

**FIG. 2**

**FIG. 3**

EP 2 207 507 B1

FIG. 4

EP 2 207 507 B1

FIG. 5

**FIG. 6**

EP 2 207 507 B1

**FIG. 7**

**FIG. 8**

EP 2 207 507 B1

**FIG. 9**

EP 2 207 507 B1

**FIG. 10**

EP 2 207 507 B1

FIG. 11

FIG. 12

FIG. 13

EP 2 207 507 B1

**FIG. 14**

EP 2 207 507 B1

**FIG. 15**

EP 2 207 507 B1

**FIG. 16**

**EP 2 207 507 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4466953 A
- US 4470962 A
- US 4588580 A
- US 4626539 A
- US 5006342 A
- US 5186939 A
- US 5310559 A
- US 5474783 A
- US 5656286 A
- US 5762952 A
- US 5948433 A
- US 5985317 A
- US 5958446 A
- US 5993849 A
- US 6024976 A
- US 6063399 A
- US 6139866 A
- US 2003002682 A
- US 20050208117 A
- US 2002119187 A
- US 20040234584 A
- US 4704282 A
- US 4725439 A
- US 4867982 A
- US 4908027 A
- US 5004610 A
- US 5152997 A
- US 5164190 A
- US 5342623 A
- US 5344656 A
- US 5364630 A
- US 5462745 A
- US 5633008 A
- US 6165497 A
- US 5693335 A
- US 5785991 A
- US 5843468 A
- US 5882676 A
- US 6004578 A

### Non-patent literature cited in the description

- Physicians Desk Reference. 2004, 1751-1755
- **SATHYAN et al.** Evaluation of the bioequivalence of two transdermal fentanyl systems following single and repeat applications. *Current Medical Research and Opinion,* 2005, vol. 21 (12), 1961-1968
- Acrylic Adhesives. **SATAS.** Handbook of pressure-Sensitive Adhesive Technology. Van Nostrand Reinhold, 1989, 396-456